(19) Europäisches Patentamt
European Patent Office
Office européen des brevets

(11) Publication number: **0 105 651**
**A1**

(12) # EUROPEAN PATENT APPLICATION

(21) Application number: 83305422.4

(22) Date of filing: 15.09.83

(51) Int. Cl.³: C 07 D 309/12
//C07C177/00

(30) Priority: 17.09.82 JP 160858/82

(43) Date of publication of application:
18.04.84 Bulletin 84/16

(84) Designated Contracting States:
AT BE CH DE FR GB IT LI LU NL SE

(71) Applicant: ONO PHARMACEUTICAL CO., LTD.
No. 14, Doshomachi 2-chome Higashi-ku
Osaka-shi Osaka(JP)

(72) Inventor: Konishi, Yoshitaka
3-3-602, Miyano-cho
Takatsuki-shi Osaka(JP)

(72) Inventor: Miyake, Hajimu
14-8, Nasahara-motomachi
Takatsuki-shi Osaka(JP)

(74) Representative: Bentham, Stephen et al,
J.A. Kemp & Co. 14 South Square Gray's Inn
London WC1R 5EU(GB)

(54) **Bicyclo(3.3.0)octane derivatives.**

(57) Bicyclo[3.3.0]octane derivatives of the formula;

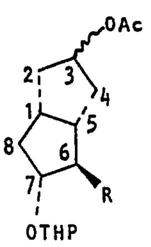

VII

[wherein Ac represents an acetyl group, THP represents a tetrahydropyran-2-yl group and R represents a formyl group or a group of the formula:

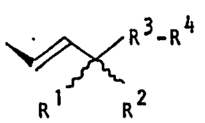

VIII

(in which one of $R^1$ and $R^2$ represents a hydroxy group and the other represents a hydrogen atom, or $R^1$ and $R^2$ together represent an oxo group, $R^3$ represents a single bond or a straight- or branched-chain alkylene group of 1 to 5 carbon atoms and $R^4$ represents a straight- or branched-chain alkyl group of 1 to 8 carbon atoms, a straight- or branched-chain alkenyl or alkynyl group of 2 to 8 carbon atoms, a cycloalkyl group of 4 to 7 carbon atoms either unsubstituted or substituted by at least one straight- or branched-chain alkyl group of 1 to 8 carbon atoms or represents a phenyl group or phenoxy group either unsubstituted or substituted by at least one halogen atom, trifluoromethyl group or straight- or branched-chain alkyl group of 1 to 4 carbon atoms, and the double bond in the formula VIII is E; with the proviso that when $R^3$ represents a single bond, $R^4$ does not represent a substituted or unsubstituted phenoxy group)] are useful as important intermediates for the preparation of 6,9-methano-$PGI_2$ and derivatives thereof.



Croydon Printing Company Ltd.

"BICYCLO[3.3.0]OCTANE DERIVATIVES"

This invention relates to new bicyclo[3.3.0] octane derivatives. More particularly, it relates to bicyclo[3.3.0]octane derivatives useful as important intermediates for the preparation of 6,9-methano-$PGI_2$ analogues, especially the (5E)-isomers thereof, and to processes for the preparation thereof.

(5E)-6,9-methano-$PGI_2$ (i.e. carbacyclin) is a compound having the following structural formula:

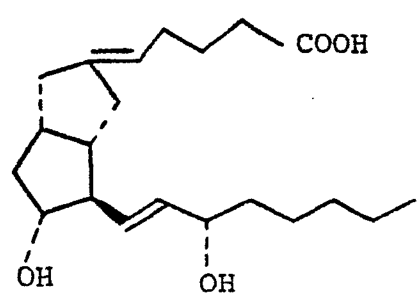

I

Carbacyclin has the same pharmacological properties as $PGI_2$ has, and further has high stability as compared with $PGI_2$, and, therefore, is expected to be developed as a medicine.

There have been filed many patent applications relating to (5E)-6,9-methano-$PGI_2$ derivatives. In particular (5E)-15-cyclopentyl-16,17,18,19,20-pentanor-6,9-methano-$PGI_2$ having the following structural formula:

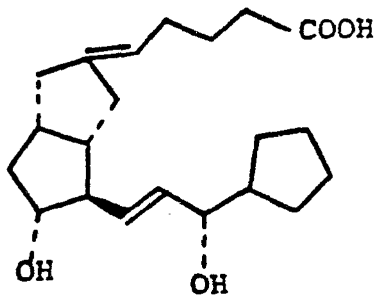

II

has a potent inhibitory activity on platelet aggregation and an excellent protective activity on brain anoxia, and, therefore, may be useful in the treatment of circulatory diseases and in the treatment of anoxia of brain cells (see British Patent No. 2017699B (Derwent No. 73954B) and German Patent Application No. 3315356.6).

There have also been many proposals relating to processes for the preparation of (5E)-6,9-methano-$PGI_2$ and derivatives thereof. However, these processes are not necessarily advantageous for commercial mass-production, and there is still great room for improvement.

In the preparation of (5E)-6,9-methano-$PGI_2$ and derivatives thereof, the known processes which are generally used involve the introduction of upper and lower side chains into various specific bicyclo[3.3.0]-octane derivatives (represented, as a "skeleton" compound, by general formula III) by using Wittig reactions as shown in the scheme below. Therefore, the bicyclo[3.3.0]-octane derivative of the formula III is considered to be a very important intermediate for the preparation of

(5E)-6,9-methano-$PGI_2$ and derivatives thereof, and thus it is important to use a bicyclo[3.3.0]octane derivative of the formula III which permits good chemical yields to be obtained and allows ease of treatment in commercial mass-production. The values of symbols X and Y which appear in the scheme are given in the following discussion of specific intermediates which have hitherto been described: X represents various specific carbonyl-protecting groups or groups which can be converted to introduce a carbonyl group, Y represents various specific hydroxy-protecting groups and Z may represent a variety of substituents. It is to be understood that not all compounds embraced generally by formula III are known: the formula is used to represent certain specific known intermediate compounds which are defined and discussed in more detail hereinafter.

Scheme

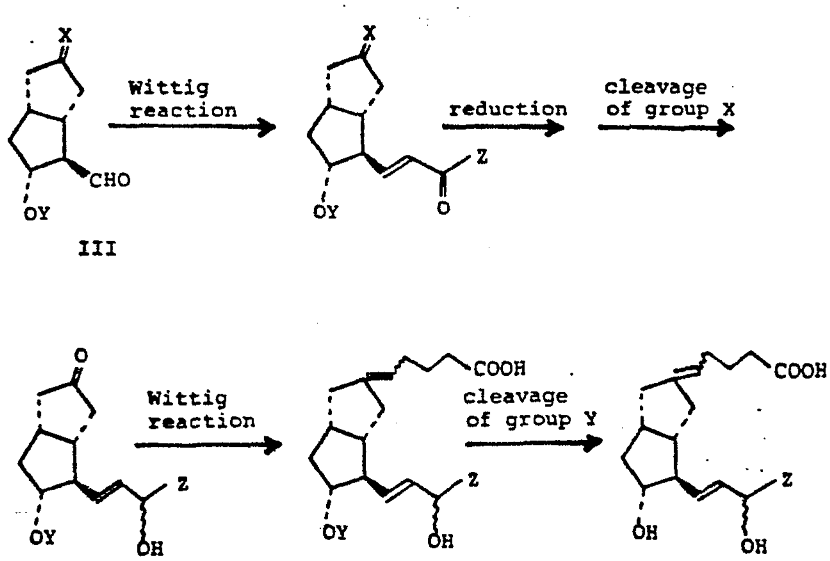


III

As bicyclo[3.3.0]octane derivatives of the formula III, several specific compounds which have various and different carbonyl-protecting groups (X) and of hydroxy-protecting groups (Y), have been proposed.

For example, in the specification of British Patent No. 2017699B (Derwent No. 73954B), there is disclosed a process which comprises subjecting a compound of the formula:

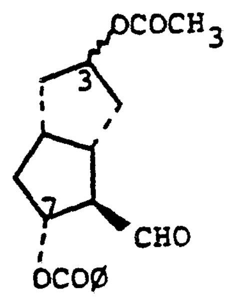

IV

(wherein Ø represents a phenyl group) to a first Wittig reaction to introduce a lower side chain, converting the acetoxy group attached to the 3-position into a hydroxy group and oxidising the hydroxy group produced to an oxo group to form a ketone, and then subjecting the compound obtained to a second Wittig reaction to introduce an upper side chain.

Furthermore, it is disclosed that the compound of the formula IV may be prepared by oxidizing a compound of the formula:

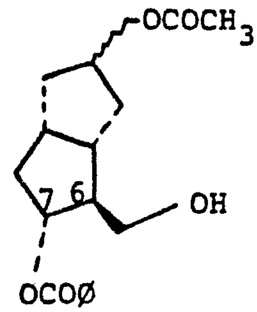

V

wherein Ø is as hereinbefore defined. However, in such an oxidation (particularly an oxidation using a base such as pyridine), it has been confirmed that a compound formed from the compound of the formula V by elimination of the benzoyl group at the 7-position to form a double bond between $C_6$ and $C_7$ positions is formed as by-product in a high proportion of 10 - 50%. Therefore, it can not be said that the compound of the formula IV having a hydroxy group protected by a benzoyl group at the 7-position, is an intermediate suitable for commercial mass-production.

In the specification of the European Patent Publication No. 11591 (Derwent No. 32903C), there is disclosed a process which comprises subjecting a compound of the formula:

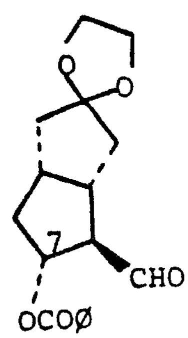

VI

wherein Ø is as hereinbefore defined,

to a first Wittig reaction to introduce a lower side chain, then cleaving the ketal to form a ketone, and then introducing an upper side chain by a similar reaction.

Furthermore, it is described that the compound of the formula VI may be prepared by the series of reactions depicted below, wherein Ø is as hereinbefore defined:

Scheme

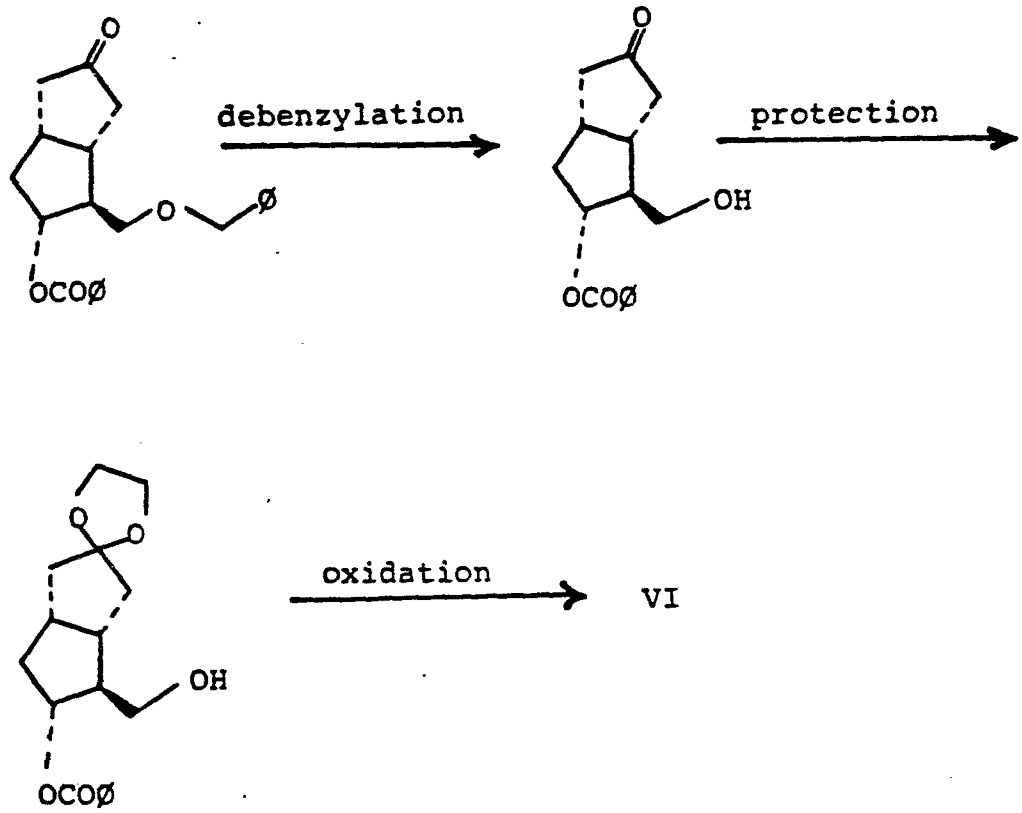

However, the catalyst presently used for the debenzylation reaction by hydrogenolysis, is generally palladium on carbon, but it is extremely dangerous to use a large quantity of palladium on carbon in commercial mass-production. In addition, the ketal group protecting the ketone group is liable to be cleaved and there is a further possibility of the ketal being cleaved in the course of subsequent reaction steps. Another disadvantage is that the benzoyl group is cleaved in the oxidation step (the hydroxy group at the 7-position is protected by a benzoyl group, as in the compound of the formula IV). From the above view point, the intermediate of the formula VI also is not a compound suitable for commercial mass-production.

In addition to the specific compounds mentioned above, various other intermediates of the formula III have been proposed [see British Patent Publication Nos. 2012265A (Derwent No. 54825B), 2013661A (Derwent No. 56290B), 2014143A (Derwent No. 59839B) and 2019847A (Derwent No. 66338B)], but all of them have some disadvantage from the view point of ease of use, chemical yield and costs.

A suitable intermediate should have protecting groups which are easily cleaved in a cleavage reaction but which otherwise remain attached and unaffected in other reactions and should not give rise to side-reactions.

As a result of research and experimentation to discover a novel intermediate of the formula III which may be converted to a desired (5E)-6,9-methano-$PGI_2$ or analogue thereof with safety, low cost, ease of treatment and high chemical yield, and further without producing side-reactions, it has been discovered that a new intermediate of the formula III wherein X represents an acetoxy group and Y represents a tetrahydropyran-2-yl group, is surprisingly advantageous.

The present invention accordingly provides new bicyclo[3.3.0]octane derivatives of the general formula:

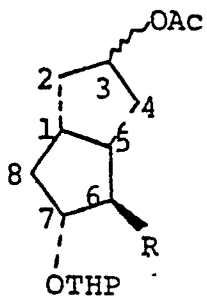

VII

[wherein Ac represents an acetyl group, THP represents a tetrahydropyran-2-yl group and R represents a formyl group or a group of the general formula:

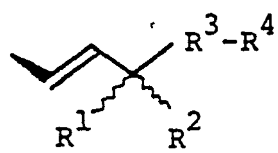

VIII

(in which one of $R^1$ and $R^2$ represents a hydroxy group and the other represents a hydrogen atom, or $R^1$ and $R^2$ together represent an oxo group, $R^3$ represents a single bond or a straight- or branched-chain alkylene group of 1 to 5 carbon atoms and $R^4$ represents a straight- or branched-chain alkyl group of 1 to 8 carbon atoms, a straight- or branched-chain alkenyl or alkynyl group of 2 to 8 carbon atoms, a cycloalkyl group of 4 to 7 carbon atoms, either unsubstituted or substituted by at least one straight- or branched-chain alkyl group of 1 to 8 carbon atoms or represents a phenyl group or phenoxy group either unsubstituted or substituted by at least one halogen atom, trifluoromethyl group or straight- or branched-chain alkyl

group of 1 to 4 carbon atoms, and the double bond in formula VIII is E; with the proviso that when $R^3$ represents a single bond, $R^4$ does not represent a substituted or unsubstituted phenoxy group)], which are useful as intermediates in the preparation of 6,9-methano-$PGI_2$ and derivatives thereof.

In the above structural formulae and in the other structural formulae in this specification, according to the generally accepted nomenclature, the broken line indicates that the substituent attached thereto is behind the ring plane, i.e. is of the α-configuration, the bold line ◀ indicates that the substituent attached thereto is in front of the ring plane, i.e. is of the β-configuration, and the wavy line ∿ indicates that the substituent attached thereto is of the α-configuration or the β-configuration or a mixture thereof.

The present invention is concerned with all compounds of general formula VII in the optically active "natural" form or its enantiomeric form, or mixtures thereof, more particularly the racemic form, consisting of equimolecular mixtures of the optically active "natural" form and its enantiomeric form.

As will be apparent to those skilled in the art, the compounds depicted in general formula VII have at least five centres of chirality, those five centres of chirality being at the C-1, C-3, C-5, C-6 and C-7 carbon atoms. Still further centres of chirality may occur when one of $R^1$ and $R^2$ in the general formula VIII represents

a hydroxy group and the other represents a hydrogen atom, and when the grouping $-R^3-R^4$ in the general formula VIII contains a branched-chain alkylene group or represents a branched-chain alkyl group. The presence of chirality leads, as is well known, to the existence of isomerism. However, the compounds of general formula VII all have a configuration that the substituent groups attached to the ring carbon atoms in the positions identified as 1 and 5 are cis with respect to each other and that the substituent groups attached to the ring carbon atoms in the positions identified as 5 and 6 are trans with respect to each other. Accordingly, all isomers of general formula VII and mixtures thereof which have those substituent groups attached to the ring carbon atoms in positions 1 and 5 in the cis-configuration, those attached in positions 5 and 6 in the trans-configuration and the acetyl group as depicted in the 3-position and the tetrahydropyran-2-yl group as depicted in the 7-position are to be considered within the scope of general formula VII.

In the general formula VIII, as the alkylene group of 1 to 5 carbon atoms represented by $R^3$ there may be mentioned methylene, ethylene, trimethylene, tetramethylene, and pentamethylene and isomers thereof. $R^3$ preferably represents a single bond or a methylene or ethylene group.

In the general formula VIII, as the alkyl group of 1 to 8 carbon atoms or alkenyl or alkynyl group of 2 to 8 carbon atoms represented by

$R^4$, there may be mentioned methyl, ethyl, propyl, butyl, pentyl, hexyl, heptyl, and octyl and isomers thereof and the corresponding unsaturated groups containing 2 to 8 carbon atoms, having a double bond or a triple bond therein; butyl, pentyl or hexyl groups optionally having a double bond or a triple bond therein and either unsubstituted or substituted by one or two methyl or ethyl groups are preferred.

In the general formula VIII, as the substituted or unsubstituted cycloalkyl group represented by $R^4$, there may be mentioned cyclobutyl, cyclopentyl, cyclohexyl, cycloheptyl and cyclobutyl, cyclopentyl, cyclohexyl and cycloheptyl groups substituted by one or more methyl, ethyl, propyl, butyl, pentyl, hexyl, heptyl or octyl groups; cyclobutyl, cyclopentyl or cyclohexyl groups either unsubstituted or substituted by one methyl, ethyl, propyl or butyl group are preferred.

In the general formula VIII, as the substituted or unsubstituted phenyl or phenoxy group represented by $R^4$, there may be mentioned phenyl and phenoxy groups, and phenyl and phenoxy groups substituted by one or more atoms or groups selected from fluorine and chlorine atoms, and trifluoromethyl, methyl, ethyl, propyl or butyl groups; phenyl or phenoxy groups either unsubstituted or substituted by one chlorine atom, trifluoromethyl group, methyl group or ethyl group are preferred.

In the general formula VIII, as preferred groupings $R^3-R^4$, there may be mentioned butyl, pentyl, 1-methylpentyl, 2-methylpentyl, 3-methylpentyl, 1,1-dimethylpentyl, 1-ethylpentyl, 2-ethylpentyl, hexyl, 1-methylhexyl, 2-methylhexyl, 1-ethylhexyl, 2-ethylhexyl, heptyl, 2-methylheptyl, 2-ethylheptyl, 4-methylpent-3-enyl, 2,4-dimethylpent-3-enyl, pent-3-ynyl and 1-methylpent-3-ynyl; cyclobutyl, 1-propylcyclobutyl, 1-butylcyclobutyl, 3-ethylcyclobutyl, 3-propylcyclobutyl, cyclopentyl, cyclopentylmethyl, 2-cyclopentylethyl, 3-ethylcyclopentyl, 3-propylcyclopentyl, 3-butylcyclopentyl, cyclohexyl, cyclohexylmethyl, 2-cyclohexylethyl, 4-methylcyclohexyl, 4-ethylcyclohexyl, 4-propylcyclohexyl and 4-butylcyclohexyl; and also benzyl, 2-phenylethyl, 4-methylbenzyl, 4-ethylbenzyl, phenoxymethyl, 2-phenoxyethyl, 3-chlorophenoxymethyl, 4-chlorophenoxymethyl, 3-trifluoromethylphenoxymethyl, 4-trifluoromethylphenoxymethyl, 4-methylphenoxymethyl and 4-ethylphenoxymethyl; cyclopentyl is especially preferred.

Further, in the general formula VIII, the preferred configuration of the hydroxy group is the α-configuration, when one of $R^1$ and $R^2$ represents a hydroxy group and the other represents a hydrogen atom.

Compounds of general formula VII wherein the grouping $R^3-R^4$ represents a cycloalkyl group of 4 to 7 carbon atoms either unsubstituted or substituted by at least one straight- or branched-chain alkyl group of 1 to 8 carbon atoms, especially a cyclopentyl group, are especially preferred.

According to a feature of the present invention,

compounds of the general formula VII wherein R represents the group of the general formula VIII, in which one of $R^1$ and $R^2$ represents a hydroxy group and the other represents a hydrogen atom, i.e. compounds of the general formula:

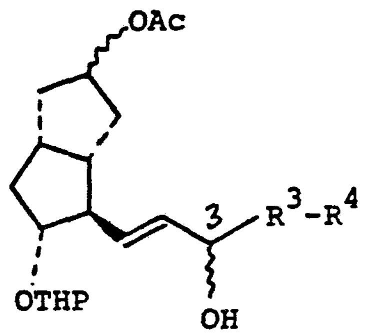

VIIA

(wherein the various symbols are as hereinbefore defined) may be prepared by reducing a compound of the general formula VII wherein R represents the group of the general formula VIII, in which $R^1$ and $R^2$ together represent an oxo group, i.e. a compound of the general formula:

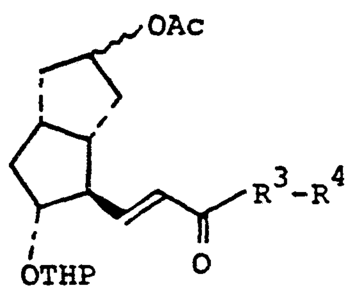

VIIB

wherein the various symbols are as hereinbefore defined,

The reduction to convert the oxo group to a hydroxy group may be carried out by using any suitable reducing reagent such as sodium borohydride, potassium borohydride, lithium borohydride, zinc borohydride, lithium tri-tert-butoxyaluminium hydride, lithium

trimethoxyaluminium hydride, sodium cyanoborohydride, potassium tri-sec-butylborohydride, lithium aluminium hydride-quinine complex, (-)-isobornyloxymagnesium iodide in an inert organic solvent, e.g. an alkanol containing from 1 to 4 carbon atoms such as methanol, ethanol or isopropanol, or an ether such as tetrahydrofuran, dioxan or 1,2-dimethoxyethane, or a mixture of two or more such solvents, at a temperature from -78°C to ambient. Preferably, the reduction is effected using diisobornyloxyaluminiumisopropoxide (described in our Japanese Patent Kokai No. 54-76552), or a diisobutyl(alkyl-substituted or unsubstituted) phenoxyaluminium [described in our Japanese Patent Kokai No. 54-154739 and J. Org. Chem., 44, 1363(1979)], or a lithium 1,1'-binaphthyl-2,2'-dioxyaluminium hydride [described in J. Amer. Chem. Soc., 101, 5843(1979)]. The product thus obtained is a mixture of isomers in which the 3-hydroxy group is in α- or β-configuration and the mixture, if desired, is separated by conventional means, for example, by thin layer, column or high-speed liquid chromatography on silica gel to give the desired isomer of general formula VIIA.

According to a further feature of the invention compounds of the general formula VIIB may be prepared by subjecting to the Wittig reaction a compound of the general formula VII wherein R represents a formyl

group, i.e.by reacting a compound of the general formula:

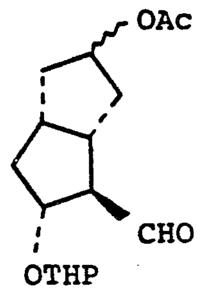

VIIC

(wherein the various symbols are as hereinbefore defined) with a sodium derivative of a dialkyl phosphonate of the general formula:

$$(R^5O)_2\underset{\underset{O}{\|}}{P}CH_2\underset{\underset{O}{\|}}{C}-R^3-R^4 \qquad \text{IX}$$

(wherein $R^5$ represents an alkyl group of 1 to 4 carbon atoms, preferably methyl or ethyl, and the other symbols are as hereinbefore defined), or with a phosphorane compound of the general formula:

$$(R^6)_3P{=}CH\underset{\underset{O}{\|}}{C}-R^3-R^4 \qquad \text{X}$$

(wherein $R^6$ represents a phenyl group unsubstituted or substituted by at least one alkyl group of 1 to 4 carbon atoms, preferably phenyl, or represents an alkyl group of 1 to 6 carbon atoms, preferably butyl or hexyl or represents a cyclohexyl group, and the other symbols are as hereinbefore defined). The sodium derivative of the dialkyl phosphonate of the general formula IX may be prepared by the reaction of the dialkyl phosphonate and

sodium hydride.

The Wittig reaction is described in "Organic Reactions", Volume 14, Chapter 3 (1965), John Wiley & Sons, Inc. (USA). The reaction may be effected in an inert organic solvent, e.g. an ether such as diethyl ether, tetrahydrofuran, dioxan or 1,2-dimethoxyethane, a hydrocarbon such as benzene, toluene, xylene or hexane, a dialkyl sulphoxide such as dimethyl sulphoxide, a dialkylformamide such as N,N-dimethylformamide, a halogenated hydrocarbon such as methylene chloride or chloroform, or an alkanol containing from 1 to 4 carbon atoms such as methanol, or ethanol, or a mixture of two or more of them, at a temperature from -78°C to the reflux temperature of the reaction mixture.

Dialkyl phosphonates of the general formula IX and phosphorane compounds of the general formula X are well known, or may easily be prepared by methods known *per se*. By the term "methods known *per se*" as used in this specification is meant methods heretofore used or described in the chemical literature.

Bicyclo[3.3.0]octane derivatives of the general formula VIIC may be prepared by the series of reactions depicted schematically below in Scheme A, wherein Ø represents a phenyl group and the other symbols are as hereinbefore defined. The preparation of the derivatives of general formula VIIC from the compounds of general formula XVI constitutes a feature of the present invention.

Scheme A

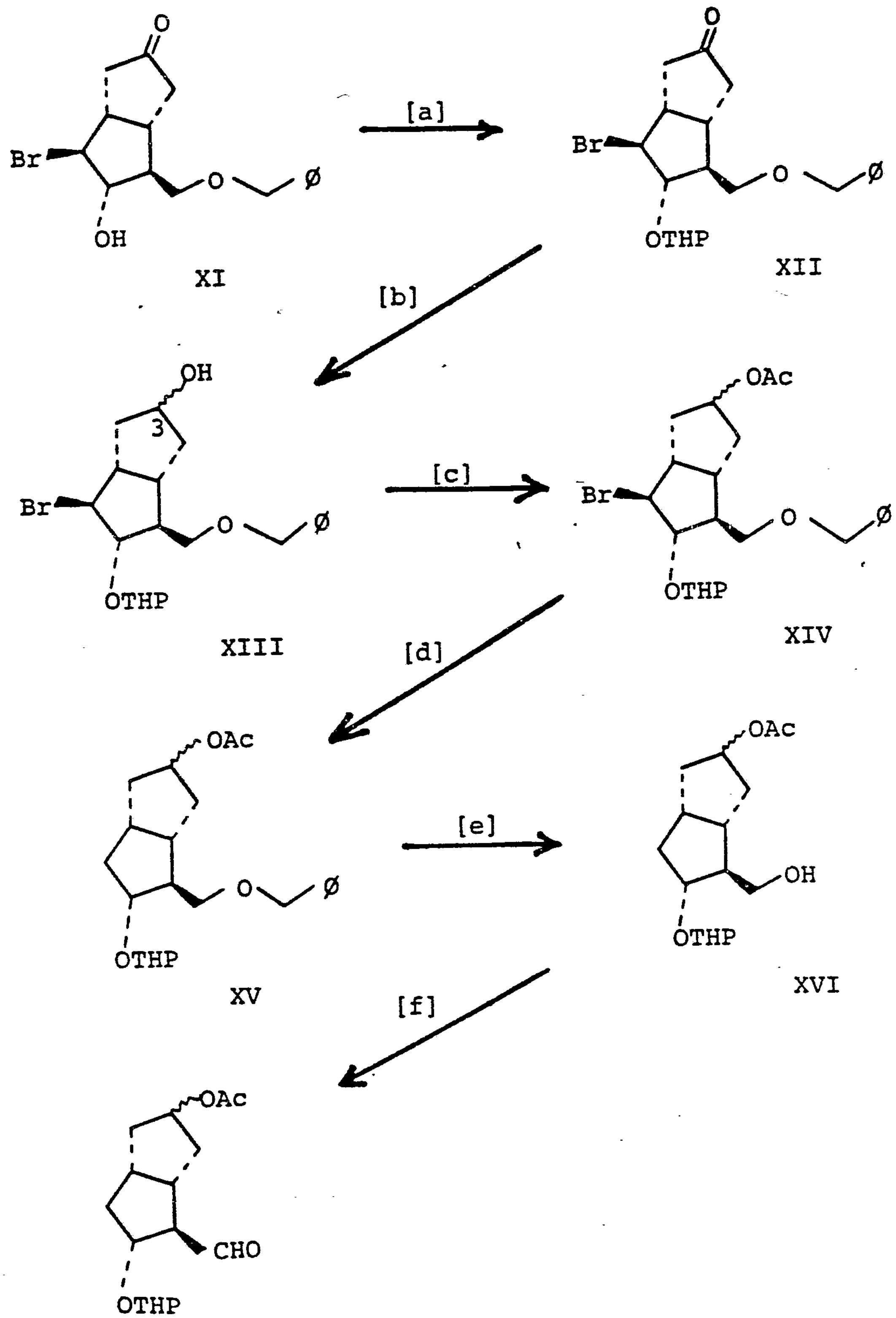


XI

XII

XIII

XIV

XV

XVI

VIIC

All of the individual reaction steps in Scheme A may be conducted by methods known *per se*, and suitable reaction conditions are described in the specification of the British Patent No. 2017699B (Derwent No. 73954B). For example, step [a], the protection of the hydroxy group by the tetrahydropyran-2-yl group, may be conducted by using 2,3-dihydropyran in an inert organic solvent such as methylene chloride in the presence of a condensing agent, e.g. p-toluenesulphonic acid or sulphuric acid at a temperature from ambient to 30°C, preferably at ambient. The step [b], reduction, may be conducted by means heretofore mentioned for the conversion of compounds of general formula VIIB to those of general formula VIIA: suitably it is effected by using sodium borohydride in methanol at a temperature lower than 0°C. The product of the general formula XIII thus obtained is a mixture of isomers in which the 3-hydroxy group is in α- or β-configuration. However the separation of such mixture is unnecessary because the 3-hydroxy group is converted again into an oxo group in a subsequent reaction step. The step [c], acetylation, may be conducted by using acetic anhydride in the presence of a tertiary amine such as pyridine or triethylamine in an inert organic solvent such as methylene chloride, or in the absence of the solvent at room temperature. The step [d], debromination, may be conducted by gentle refluxing

using tributyltin hydride in the presence of α,α'-azobisisobutyronitrile in an inert organic solvent such as benzene, toluene or ethyl acetate. The step [e], cleavage of the benzyl group, may be generally conducted by hydrogenolysis in the presence of a hydrogenation catalyst such as palladium on carbon, palladium black or platinum dioxide, in an inert organic solvent, for example a lower alkanol such as methanol or ethanol at room temperature at normal or elevated pressure, for example at a hydrogen pressure from atmospheric to 15 $kg/cm^2$. Suitably the cleavage may also be conducted by using Raney-Nickel catalyst in a lower alkanol such as methanol or ethanol under an atmosphere of an inert gas, for example under an atmosphere of argon gas at a temperature from ambient to the reflux temperature of the reaction mixture. For the purpose of commercial mass-production it is of great significance that Raney-Nickel can be used as catalyst from a view point of safety. The step [f], oxidation, may be carried out as described in detail in, for example,

(a) "Synthetic Organic Chemistry III, Organic Synthesis 1", pp. 176-206 (compiled by Tetsuji Kametani and published by Nankodo (Japan) on Aug. 1, 1976) or

(b) "Compendium of Organic Synthetic Methods", vol. 1, vol. 2. and vol. 3, section 48 or 168 [published by John Wiley & Sons, Inc. (USA) in 1971, 1974 and 1977, respectively].

The oxidation is preferably carried out under mild neutral conditions using, for example, sulphuric anhydride-pyridine complex [see J. Amer. Chem. Soc., 89, 5505 (1967)], chromium trioxide-pyridine complex (for example, Collins' reagent), Jones' reagent or chromic acid solution (prepared from chromium trioxide, manganese sulphate, sulphuric acid and water), or oxalyl chloride and dimethylsulphoxide (i.e. Swern oxidation). The oxidation using sulphuric anhydride-pyridine complex may be carried out in dimethyl sulphoxide in the presence of triethylamine at room temperature. The Collins oxidation may be conducted in a halogenated hydrocarbon such as chloroform, methylene chloride or carbon tetrachloride at a temperature from room temperature to 0°C, preferably at 0°C. The Jones oxidation may be conducted at a temperature of not higher than room temperature. The Swern oxidation may be conducted by reaction in a halogenated hydrocarbon such as chloroform or methylene chloride at a temperature from -50°C to -60°C, and then treatment with triethylamine.

In the oxidation of the compound of formula XVI a side-reaction to form a double bond between the $C_6$ and $C_7$ positions (which may occur as hereinbefore described in the compound of formula V) does not occur because the hydroxy group at the 7-position is protected not by a benzoyl group but by a tetrahydropyran-2-yl group.

In the sequence of the reaction steps illustrated by the above scheme A, the compound of the formula XI employed as the starting material is known per se, and is described in the specification of the British Patent No. 2017699B (Derwent No. 73954B).

The bicyclo[3.3.0]octane compounds of the general formula VIIA of the present invention may be converted to pharmacologically active 6,9-methano-$PGI_2$ analogues of the general formula:

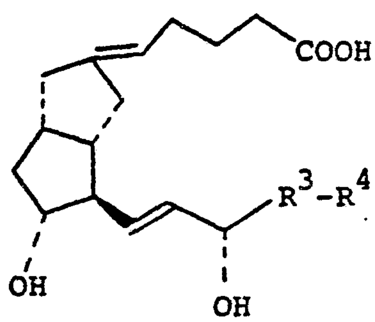

I'

(wherein the various symbols are as hereinbefore defined) by the series of reactions depicted schematically below in Scheme B, wherein BMS represents a tert-butyldimethylsilyl group and the other symbols are as hereinbefore defined.

Scheme B

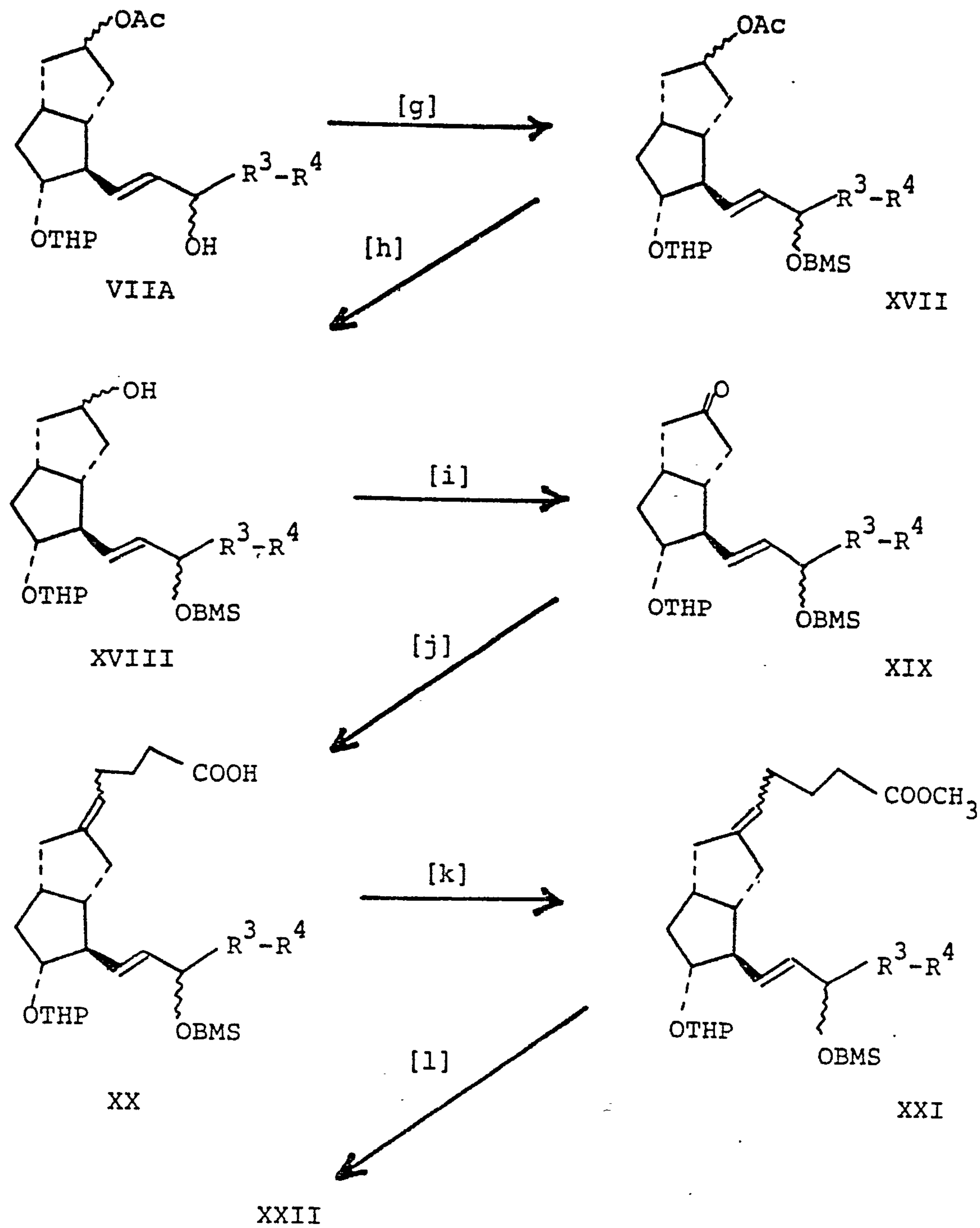


VIIA

XVII

XVIII

XIX

XX

XXI

XXII

Scheme B (continued)

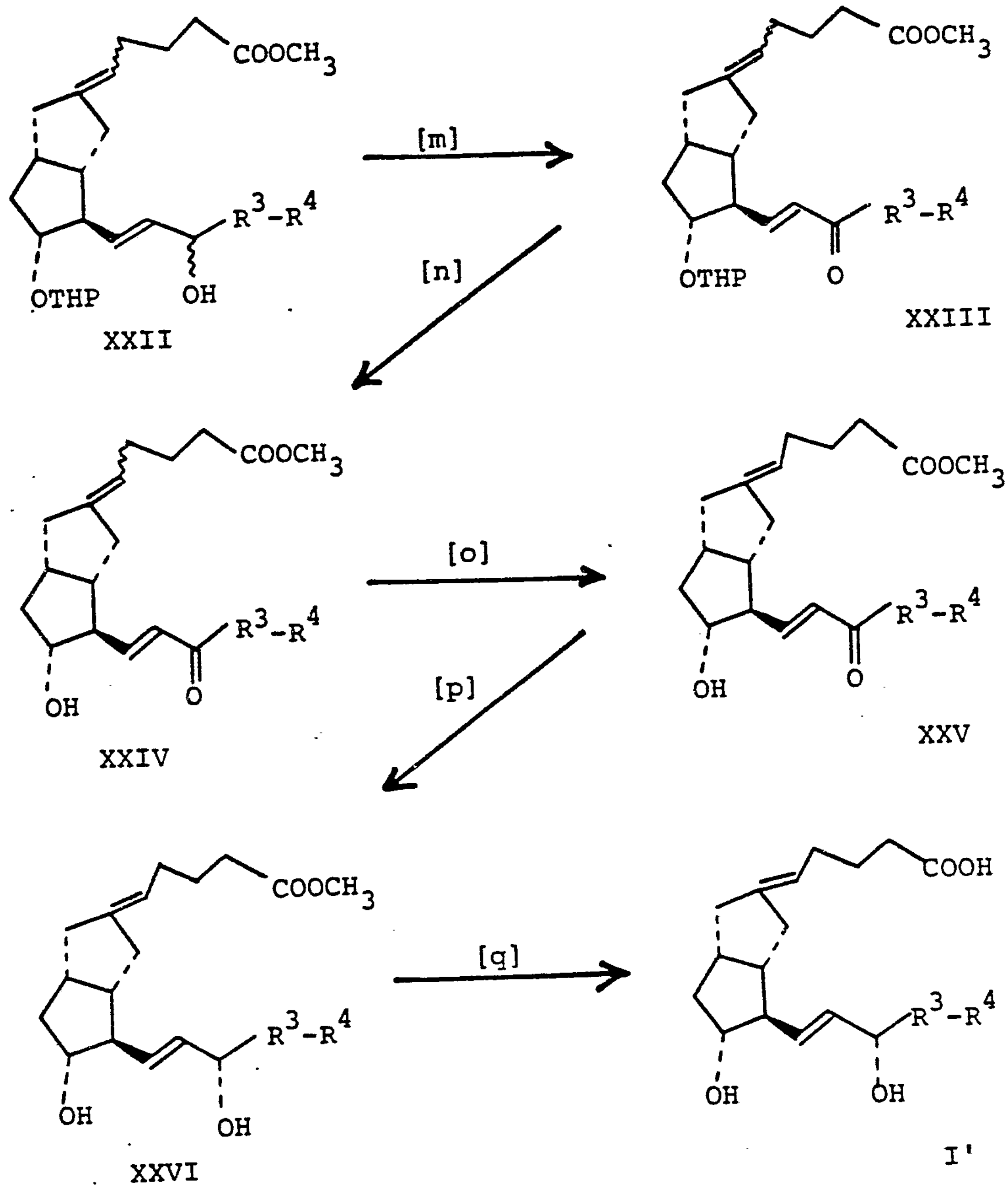


XXII

XXIII

XXIV

XXV

XXVI

I'

All of the individual reaction steps in Scheme B may be conducted by methods known per se, and suitable reaction conditions are described in the specification of the British Patent No. 2017699B (Derwent No. 73954B). For example, step [g], the protection of the hydroxy group by the tert-butyldimethylsilyl group, may be conducted by using tert-butyldimethylsilyl chloride in an inert organic solvent such as dimethylformamide in the presence of a base such as imidazole at room temperature. The step [h], deacetylation, may be conducted by using potassium carbonate in methanol at room temperature. The step [i] may be conducted as described for step [f]. The step [j], the Wittig reaction, may be conducted by reaction with (4-carboxybutylidene)-triphenylphosphorane of the formula: $\emptyset_3P{=}CH{-}(CH_2)_3{-}COOH$ (in which Ø is as hereinbefore defined) in toluene at room temperature. The phosphorane compound may be obtained by the reaction of (4-carboxybutyl)triphenylphosphonium bromide with potassium tert-butoxide. The step [k], esterification, may be conducted by using a solution of diazomethane in diethyl ether, in ethyl acetate at room temperature. The step [l], cleavage of the tert-butyldimethylsilyl group, may be conducted by using tetrabutylammonium fluoride ($n{-}Bu_4N^+F^-$) in tetrahydrofuran at room temperature. The step [m], oxidation, may be conducted as described for the step [f]. The step [n], cleavage of the tetrahydropyran-2-yl group, may be conducted by using a mixture of tetrahydrofuran, acetic acid and water,

a mixture of p-toluenesulphonic acid and methanol, or a mixture of dilute hydrochloric acid and tetrahydrofuran at room temperature or under heating. The step [o], separation of (5E)-isomer from (5Z)-isomer, may be conducted by conventional means, for example, by thin layer, column or high-speed liquid chromatography on silica gel. The step [p] may be conducted by means heretofore mentioned for the conversion of compounds of general formula VIIB to those of general formula VIIA. The step [q], saponification, may be conducted by using an aqueous solution of potassium hydroxide in ethanol. Compounds of the general formulæ XXVI and I' may, if desired, be purified by recrystallization from a suitable solvent.

In the sequence of the reaction steps described above, the separation of (5E)-isomer from (5Z)-isomer may also be carried out after introduction of the α-chain by the Wittig reaction (i.e. purification of the compound of the general formula XX), or as final product (i.e. purification of the compound of the general formula I') in good yield. The separation of (5E)-isomer in the form of the compound of the general formula XXIV having an enone group ( 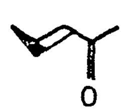 ) gives the highest yield. The proportion of (5E)-isomer to (5Z)-isomer is about 5 to 1 when the separation is conducted in the step [o].

As described above, the separation of (5E)-isomer from (5Z)-isomer may also be conducted on the compound of the general formula XX or on the compound of the general formula I'. In such case, it is not necessary to use different protecting groups at the 11- and 15-positions, the sequence of the reaction steps then being different from that shown in Scheme B. It is then possible to decrease the number of reaction steps from the compound of the general formula VIIA to the final product. Furthermore, the separation of (5E)-isomer may be carried out in the form of a dicyclohexylamine salt of the final product (see Japanese Patent Kokai No. 56-122328) as well as by conventional chromatographic separations.

When the separation is carried out after introduction of the α-chain by the Wittig reaction or on the final product, compounds of the general formula I' may be prepared from a compound of the general formula VIIA by the series of reactions depicted schematically below in Scheme C, wherein the various symbols are as hereinbefore defined.

Scheme C

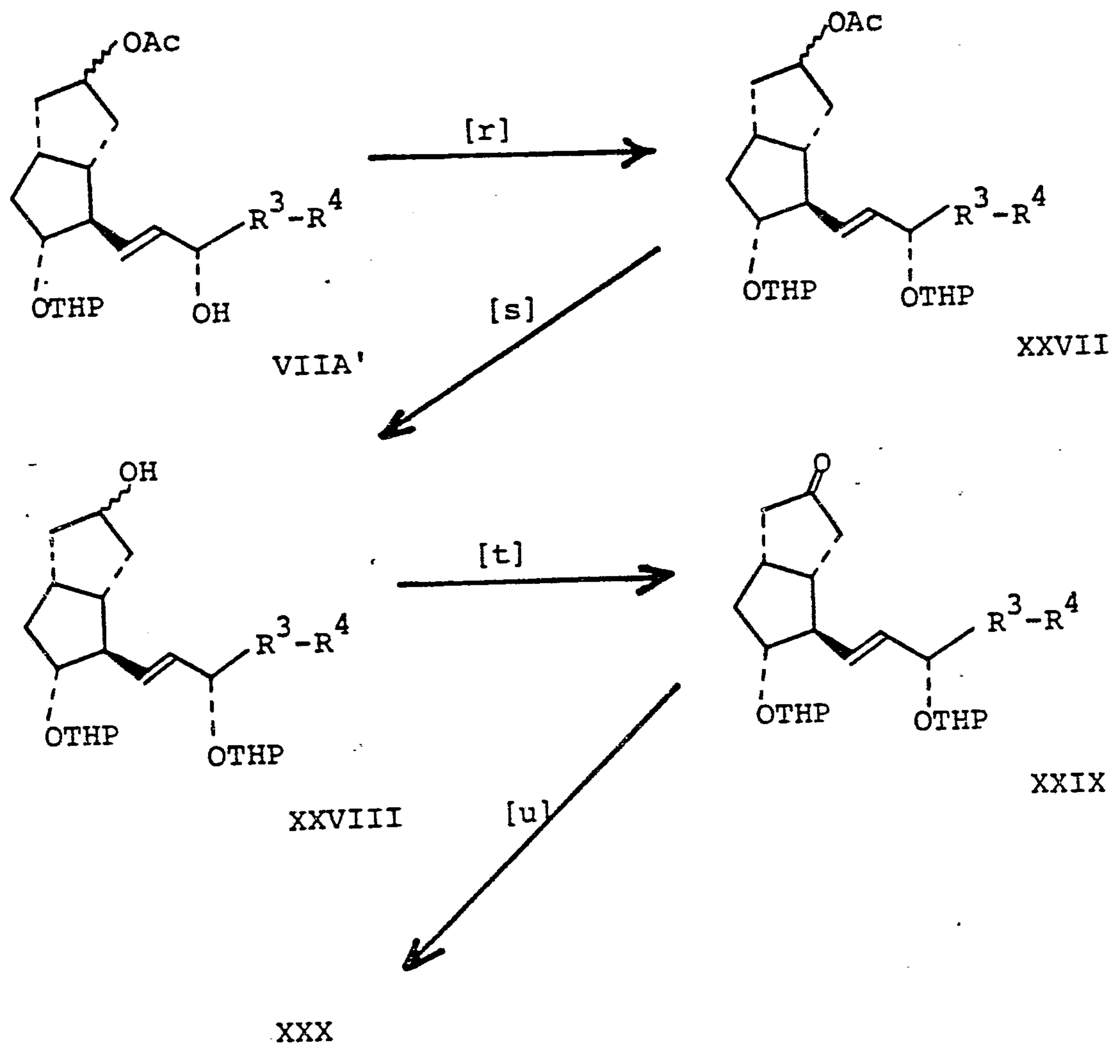


VIIA'

XXVII

XXVIII

XXIX

XXX

Scheme C (continued)

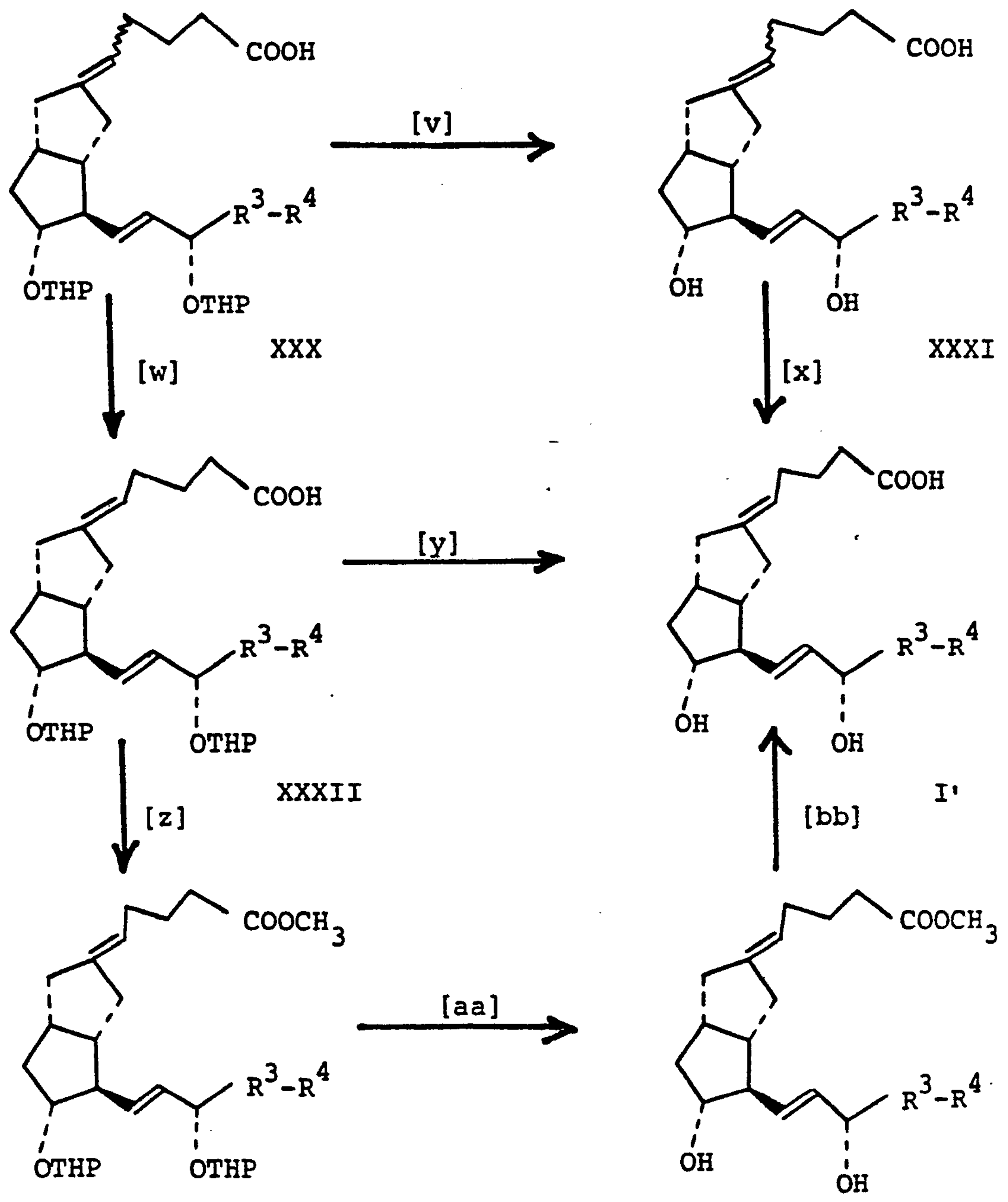


XXX

XXXI

XXXII

I'

XXXIII

XXXIV

All of the individual reaction steps in Scheme C may be conducted by methods known *per se*, and suitable conditions are described in the specification of British Patent No. 2017699B (Derwent No. 73954B). For example, step [r], protection by a THP group, may be conducted as hereinbefore described for the step [a]. The step [s], deacetylation, may be conducted as hereinbefore described for the step [h]. The step [t], oxidation, may be conducted as hereinbefore described for the step [f]. The step [u], the Wittig reaction, may be conducted as hereinbefore described for the step [j]. The steps [v], [y] and [aa], cleavage of the THP group, may be conducted as hereinbefore described for the step [n]. The steps [w] and [x], separation of (5E)-isomer and (5Z)-isomer, may be conducted as hereinbefore described for the step [o]. Recrystallization can, if desired, give (5E)-isomer in higher purity. The step [x] may be also carried out by forming a dicyclohexylamine salt, i.e., it can be conducted by forming a dicyclohexylamine salt of a compound of the general formula XXXI by using dicyclohexylamine in a suitable solvent, e.g. acetone, and then, if desired, recrystallizing the resulting salt from a suitable solvent, and thereafter converting the salt to the free carboxylic acid under acidic aqueous conditions, e.g. in an aqueous solution of potassium bisulfate, to give a compound of the general formula I' rich in

(5E)-isomer thereof. The step [z], esterification, may be conducted as hereinbefore described for the step [k], or by using methyl iodide in acetone in the presence of an alkali metal, e.g. potassium, carbonate. The step [bb], saponification, may be conducted as hereinbefore described for the step [q]. Compounds of the general formulae XXXIV and I' may, if desired, be purified by recrystallization from a suitable solvent.

The (5E)-6,9-methano-$PGI_2$ and derivatives thereof thus obtained have various useful pharmacological properties, (see British Patent No. 2017699B, and British Patent Publication Nos. 2012265A, 2013661A, 2014143A and 2019847A, and the German Patent Application No. P3315356.6).

It will be appreciated, therefore, that the new compounds of the present invention of the general formula VII, i.e. compounds of the general formula VIIA, VIIB and VIIC are useful and important intermediates for the preparation of therapeutically useful (5E)-6,9-methano-$PGI_2$ and derivatives thereof.

The use of the bicyclo[3.3.0]octane compounds of the general formula VII allows the synthesis of (5E)-6,9-methano-$PGI_2$ and derivatives thereof by the methods hereinbefore described which avoid certain disadvantages in the use of compounds of the general formula IV and VI and other bicyclo[3.3.0]octane compounds. The use of the compounds of the general formula VII avoids

the production of by-product, and the need to use a ketal group as a protecting group of a ketone, and enables debenzylation to be carried out using Raney-Nickel catalyst.

The following Reference Examples and Examples illustrate the preparation of compounds of the present invention. In the Reference Examples and Examples, 'TLC', 'NMR', 'IR' and 'Mass' represent 'Thin layer chromatography', 'Nuclear magnetic resonance spectrum', 'Infrared absorption spectrum' and 'Mass spectrum', respectively. The solvents in parentheses specified in chromatographic separations show the developing solvents used: ratios are by volume. Except when specified otherwise, infrared absorption spectra were recorded by the liquid film method and nuclear magnetic resonance spectra were recorded in deuterochloroform ($CDCl_3$) solution.

Reference Example 1

6-syn-benzyloxymethyl-7-anti-(tetrahydropyran-2-yloxy)-8-syn-bromo-cis-bicyclo[3.3.0]-octan-3-one

Under an atmosphere of argon, to a mixture of 4.7 g of 6-syn-benzyloxymethyl-7-anti-hydroxy-8-syn-bromo-cis-bicyclo[3.3.0]octan -3-one [prepared as described in the specification of the British Patent No. 2017699B (Derwent No. 73954B), Reference Example 4], 1.52 g of 2,3-dihydropyran and 50 ml of methylene chloride was added a catalytic amount of p-toluenesulphonic acid, and the mixture was stirred for 20 minutes at room temperature. To the reaction mixture was added a saturated aqueous solution of sodium bicarbonate and the mixture was extracted with ethyl acetate. The extract was washed with water and a saturated aqueous solution of sodium chloride, successively, dried over anhydrous magnesium sulphate and concentrated under reduced pressure to give ca. 6.0 g of the title compound as crude product having the following physical characteristics:

TLC (ethyl acetate:cyclohexane=1:2): Rf=0.47;

NMR: $\delta$=7.34 (5H, s), 5.00-4.65 (1H, m), 4.55 (2H, s), 4.50-3.20 (6H, m);

IR: $\nu$=3040, 2950, 2860, 1742, 1451, 1404, 1356, 1200, 1121, 1078, 1035, 968, 740, 700 $cm^{-1}$;

Mass: m/e=422($M^+$), 337, 241, 191, 151, 135, 107, 101, 91, 85.

Reference Example 2

6-syn-benzyloxymethyl-7-anti-(tetrahydropyran-2-yloxy)-8-syn-bromo-cis-bicyclo[3.3.0]octan-3αβ-ol

To a mixture of ca. 6.0 g of the bicyclo[3.3.0]-octan-3-one compound (prepared in Reference Example 1) and 65 ml of methanol were added 950 mg of sodium borohydride little by little over a period of 10 minutes at -20°C. After stirring 5 minutes, acetic acid was added thereto to decompose an excess amount of sodium borohydride and the reaction mixture was concentrated under reduced pressure to remove methanol. To the residue obtained was added water and the mixture was extracted with ethyl acetate and the extract was washed with a saturated aqueous solution of sodium bicarbonate, water and a saturated aqueous solution of sodium chloride, successively, dried over anhydrous magnesium sulphate and concentrated under reduced pressure to give ca. 6.2 g of the title compound as crude product having the following physical characteristic:

TLC (ethyl acetate:cyclohexane=1:2): Rf=0.31 and 0.36.

Reference Example 3

3αβ-acetoxy-6-syn-benzyloxymethyl-7-anti-(tetrahydropyran-2-yloxy)-8-syn-bromo-cis-bicyclo[3.3.0]octane

A mixture of ca. 6.2 g of the bicyclo[3.3.0]octan-3-ol compound (prepared in Reference Example 2), 5.2 ml of acetic anhydride and 8.8 ml of pyridine was stirred for 5 hours at room temperature and concentrated under reduced pressure. The residue thus obtained was diluted with ethyl acetate, washed with 1N hydrochloric acid, a saturated aqueous solution of sodium bicarbonate, water and a saturated aqueous solution of sodium chloride, successively, dried over anhydrous magnesium sulphate and concentrated under reduced pressure to give ca. 6.45 g of the title compound as crude product having the following physical characteristics:

TLC (ethyl acetate:cyclohexane=1:2): Rf=0.50;

NMR: δ=7.32 (5H, s), 5.22 (1H, m), 4.94 and 4.73 (1H, m), 4.51 (2H, s), 4.20-3.36 (6H, m), 2.02 (3H, s);

IR: ν=1735, 1450, 1370, 1240, 1130, 1070, 1025, 970, 905, 870, 815, 735, 695 $cm^{-1}$;

Mass: m/e=466($M^+$), 381, 215, 191, 179, 165, 151, 135, 119, 105, 101, 91, 85.

Reference Example 4

3αβ-acetoxy-6-syn-benzyloxymethyl-7-anti-(tetrahydropyran-2-yloxy)-cis-bicyclo[3.3.0]octane

Under an atmosphere of argon, a mixture of ca. 6.45 g of the 8-bromo-bicyclo[3.3.0]octane compound (prepared in Reference Example 3), 4.25 g of tributyltin hydride, 67 mg of α,α'-azobisisobutyronitrile and 60 ml of benzene was refluxed gently for 1.2 hours. The reaction mixture was concentrated under reduced pressure and the residue was purified twice by column chromatography on silica gel (eluent; a mixture of ethyl acetate and cyclohexane) to give 5.07 g of the title compound having the following physical characteristics:

TLC (benzene:ethyl acetate=4:1): Rf=0.50;

NMR: δ=7.31 (5H, s), 5.30-5.05 (1H, m), 4.70-4.43 (3H, m), 4.06-3.38 (5H, m), 2.02 (3H, s);

IR: ν=1735, 1450, 1370, 1355, 1250, 1120, 1075, 1025, 975, 910, 865, 810, 735, 695 $cm^{-1}$;

Mass: m/e=388($M^+$), 303, 286, 243, 227, 209, 195, 180, 151, 137, 120, 101, 91, 85, 79, 67.

Reference Example 5

3αβ-acetoxy-6-syn-hydroxymethyl-7-anti-(tetrahydropyran-2-yloxy)-cis-bicyclo[3.3.0]octane

To a solution of 6.25 g of sodium hydroxide in 25 ml of water were added slowly 5 g of a 50% aluminium-nickel alloy over a period of 20 minutes at 50°C and the mixture was stirred for 45 minutes at the same temperature. The supernatant solution was decanted and the residue was washed with water and ethanol, successively to give Raney-Nickel.

To Raney-Nickel thus obtained were added 20 ml of ethanol and a solution of 1.94 g of the 6-benzyloxymethyl-bicyclo[3.3.0]octane compound (prepared in Reference Example 4), in 20 ml of ethanol was added thereto all at once with gentle refluxing and the mixture was refluxed for 1.5 hours under an atmosphere of argon. The reaction mixture was filtered through celite and concentrated under reduced pressure. The residue was purified by column chromatography on silica gel (eluent; a mixture of ethyl acetate and cyclohexane) to give 1.35 g of the title compound having the following physical characteristics:

TLC (ethyl acetate:cyclohexane=1:2): Rf=0.1;

NMR: δ=5.15 (1H, m), 4.68 and 4.57 (1H, m), 4.00-3.44 (5H, m), 2.03 (3H, s);

IR: ν=3460, 1735, 1435, 1375, 1250, 1135, 1075, 1020, 980, 910, 870, 810 $cm^{-1}$;

Mass: m/e=288($M^+$), 280, 238, 225, 213, 196, 179, 155,

137, 119, 101, 85, 67.

Example 1

3αβ-acetoxy-6-syn-formyl-7-anti-(tetrahydropyran-2-yloxy)-cis-bicyclo[3.3.0]octane

Under an atmosphere of argon, to a mixture of 1.32 g of the 6-hydroxymethyl-bicyclo[3.3.0]octane compound (prepared in Reference Example 5), 3.61 ml of triethylamine and 10 ml of dimethyl sulphoxide was added dropwise a solution of 2.07 g of sulphuric anhydride-pyridine complex in 4 ml of dimethyl sulphoxide at room temperature and the mixture was stirred for 10 minutes at the same temperature. The reaction mixture was poured into 70 ml of ice-water and extracted with a mixture of ethyl acetate and diethyl ether (1:1). The extract was washed with 1N hydrochloric acid, a saturated aqueous solution of sodium bicarbonate and a saturated aqueous solution of sodium chloride, successively, dried over anhydrous magnesium sulphate and concentrated under reduced pressure to give 1.31 g of the title compound as crude product having the following physical characteristic:

TLC (ethyl acetate:cyclohexane=1:2): Rf=0.30.

Example 2

(E)-3αβ-acetoxy-6-syn-(3-oxo-3-cyclopentylprop-1-enyl)-7-anti-(tetrahydropyran-2-yloxy)-cis-bicyclo[3.3.0]-octane

Under an atmosphere of argon, to a suspension of 216 mg of sodium hydride (content: 64.1%) in 25 ml of tetrahydropyran was added dropwise a solution of 1.46 g of dimethyl 2-cyclopentyl-2-oxoethylphosphonate

[ 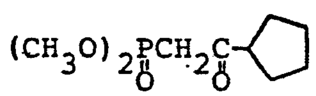 ]

in 7 ml of tetrahydrofuran at room temperature and the mixture was stirred for 30 minutes at the same temperature. To the solution thus obtained was added dropwise a solution of 1.31 g of the 6-formyl-bicyclo-[3.3.0]octane compound (prepared in Example 1) in 7 ml of tetrahydrofuran and the mixture was stirred for 20 minutes at room temperature. The reaction mixture was poured into a saturated aqueous solution of ammonium chloride and extracted with ethyl acetate. The extract was washed with water and a saturated aqueous solution of sodium chloride, successively, dried over anhydrous magnesium sulphate and concentrated under reduced pressure. The residue was purified by column chromatography on silica gel (eluent; a mixture of ethyl

acetate and cyclohexane) to give 1.5 g of the title compound having the following physical characteristics:

TLC (ethyl acetate:cyclohexane=1:2): Rf=0.40;

NMR: δ=6.91-6.70 (1H, m), 6.28-6.14 (1H, m), 5.15 (1H, m), 4.68 and 4.54 (1H, m), 4.03-3.68 (2H, m), 3.46 (1H, m), 3.08 (1H, m), 2.04 (3H, s);

IR: ν=1735, 1690, 1660, 1620, 1430, 1360, 1230, 1120, 1065, 1010, 965 $cm^{-1}$;

Mass: m/e=305, 288, 246, 229, 218, 205, 202, 177, 149, 97, 85, 69, 67.

Example 3

(E)-3αβ-acetoxy-6-syn-(3αβ-hydroxy-3-cyclopentylprop-1-enyl)-7-anti-(tetrahydropyran-2-yloxy)-cis-bicyclo[3.3.0]-octane

Proceeding as described in Reference Example 2, from 375 mg of the 6-(3-oxo-3-cyclopentylprop-1-enyl)-bicyclo[3.3.0]octane compound (prepared in Example 2), there were obtained 380 mg of the title compound as crude product, having the following physical characteristics:

TLC (ethyl acetate:cyclohexane=1:2): Rf=0.20 and 0.22;

NMR: δ=5.58 (2H, m), 5.12 (1H, m), 4.65 (1H, m), 3.94-3.65 (3H, m), 3.46 (1H, m), 2.03 (3H, s);

IR: ν=3460, 1735, 1440, 1375, 1245, 1120, 1075, 1020,

975 $cm^{-1}$;

Mass: m/e=290, 272, 248, 230, 221, 212, 196, 186, 161, 149, 136, 85, 69, 67.

Reference Example 6

(E)-3αβ-acetoxy-6-syn-[3αβ-(tert-butyldimethylsilyloxy)-3-cyclopentylprop-1-enyl]-7-anti-(tetrahydropyran-2-yloxy)-cis-bicyclo[3.3.0]octane

Under an atmosphere of argon, a mixture of 350 mg of the 6-(3αβ-hydroxy-3-cyclopentylprop-1-enyl)-bicyclo[3.3.0]octane compound (prepared in Example 3), 202 mg of tert-butyldimethylsilyl chloride, 109 mg of imidazole and 4 ml of dimethylformamide was stirred for one hour at room temperature and the reaction mixture was poured into 25 ml of ice-water. The mixture was extracted with diethyl ether and the extract was washed with 1N hydrochloric acid, a saturated aqueous solution of sodium bicarbonate, water and a saturated aqueous solution of sodium chloride, successively, dried over anhydrous magnesium sulphate and concentrated under reduced pressure to give ca. 460 mg of the title compound as crude product having the following physical characteristic:

TLC (ethyl acetate:cyclohexane=1:2): Rf=0.61.

Reference Example 7

(E)-6-syn-[3αβ-(tert-butyldimethylsilyloxy)-3-cyclopentylprop-1-enyl]-7-anti-(tetrahydropyran-2-yloxy)-cis-bicyclo[3.3.0]octan-3αβ-ol

Under an atmosphere of argon, a mixture of ca. 460 mg of the 3-acetoxy-bicyclo[3.3.0]octane compound (prepared in Reference Example 6), 122 mg of potassium carbonate and 5 ml of methanol was stirred for 15 minutes at room temperature, and further for 30 minutes at 35°C. The reaction mixture was poured into a cooled saturated aqueous solution of ammonium chloride and extracted with ethyl acetate. The extract was washed with water and a saturated aqueous solution of sodium chloride, successively, dried over anhydrous magnesium sulphate and concentrated under reduced pressure. The residue was purified by column chromatography on silica gel (eluent; a mixture of ethyl acetate and cyclohexane) to give 367 mg of the title compound having the following physical characteristic:

TLC (ethyl acetate:cyclohexane=1:2): Rf=0.39 and 0.43.

Reference Example 8

(E)-6-syn-[3αβ-(tert-butyldimethylsilyloxy)-3-cyclopentylprop-1-enyl]-7-anti-(tetrahydropyran-2-yloxy)-cis-bicyclo[3.3.0]octan-3-one

Proceeding as described in Example 1, from 350 mg of the bicyclo[3.3.0]octan-3-ol compound (prepared in Reference Example 7), there were obtained 300 mg of the title compound having the following physical characteristics:

TLC (ethyl acetate:cyclohexane=1:2): Rf=0.58;

NMR: δ=5.50 (2H, m), 4.70 (1H, m), 4.18-3.80 (3H, m), 3.50 (1H, m), 0.91 (9H, s), 0.05 (6H, m);

IR: ν=1735, 1450, 1405, 1360, 1255, 1120, 1080, 1035, 975, 915, 865, 835, 780 $cm^{-1}$;

Mass: m/e=405, 393, 361, 321, 313, 309, 293, 291, 215, 201, 177, 171, 159, 85, 65, 63.

Reference Example 9

(5EZ,13E)-(9α,11α,15αβ)-6,9-methano-11-(tetrahydropyran-2-yloxy)-15-(tert-butyldimethylsilyloxy)-15-cyclopentyl-16,17,18,19,20-pentanorprosta-5,13-dienoic acid

Under an atmosphere of argon, a mixture of 532 mg of (4-carboxybutyl)triphenylphosphonium bromide, 260

mg of potassium tert-butoxide and 4 ml of toluene was stirred for 40 minutes at 80°C. After cooling to room temperature, thereto was added a solution of 185 mg of the bicyclo[3.3.0]octan-3-one compound (prepared in Reference Example 8) in one ml of toluene at room temperature and the mixture was stirred for 1.5 hours at the same temperature. To the reaction mixture were added 4 ml of cooled water and the mixture was adjusted to pH 4 with 1N hydrochloric acid and extracted with diethyl ether. The extract was washed with water and a saturated aqueous solution of sodium chloride, successively, dried over anhydrous magnesium sulphate and concentrated under reduced pressure. The residue was purified by column chromatography on silica gel (eluent; a mixture of ethyl acetate and cyclohexane) to give 166 mg of the title compound having the following physical characteristics:

TLC (ethyl acetate:cyclohexane=1:2): Rf=0.35 and 0.41;

NMR: $\delta$=5.50 (2H, m), 5.24 (1H, m), 4.70 (1H, m), 4.00-3.70 (3H, m), 3.50 (1H, m), 0.92 (9H, s), 0.06 (6H, m);

IR: $\nu$=1710, 1450, 1250, 1115, 1075, 1030, 1015, 975, 830, 770 $cm^{-1}$;

Mass: m/e=489, 477, 471, 459, 444, 414, 405, 397, 393, 387, 375, 330, 313, 261, 159, 85, 75, 73.

Reference Example 10

(5EZ,13E)-(9α,11α,15αß)-6,9-methano-11-(tetrahydropyran-2-yloxy)-15-hydroxy-15-cyclopentyl-16,17,18,19,20-pentanorprosta-5,13-dienoic acid methyl ester

To a solution of 154 mg of the prosta-5,13-dienoic acid compound (prepared in Reference Example 9) in 3 ml of ethyl acetate was added a diethyl ether solution of diazomethane under cooling with ice until the mixture turned to pale yellow,and then the mixture was concentrated under reduced pressure to give (5EZ,13E)-(9α,11α,15αß)-6,9-methano-11-(tetrahydropyran-2-yloxy)-15-(tert-butyldimethylsilyloxy)-15-cyclopentyl-16,17,18,19,20-pentanorprosta-5,13-dienoic acid methyl ester as crude product having the following physical characteristic:

TLC (ethyl acetate:cyclohexane=1:2): Rf=0.66.

The ester thus obtained was dissolved in 3 ml of dry tetrahydrofuran and 368 mg of tetrabutylammonium fluoride were added thereto and the solution was stirred for 5 hours at room temperature. Water and ethyl acetate was added to the reaction mixture and its aqueous layer was adjusted to pH 4 with 1N hydrochloric acid and extracted with ethyl acetate. The extract was washed with water and a saturated aqueous solution of

sodium chloride, successively, dried over anhydrous magnesium sulphate and concentrated under reduced pressure. The residue was purified by column chromatography on silica gel (eluent; a mixture of ethyl acetate and cyclohexane) to give 103 mg of the title compound having the following physical characteristics:

TLC (ethyl acetate:cyclohexane=1:2): Rf=0.33 and 0.36;

NMR: δ=5.63-5.50 (2H, m), 5.21 (1H, m), 4.65 (1H, m), 3.94-3.68 (3H, m), 3.66 (3H, s), 3.45 (1H, m);

IR: ν=3470, 1735, 1435, 1245, 1200, 1160, 1120, 1075, 1010, 975, 915, 865, 810 $cm^{-1}$;

Mass: m/e=428, 384, 344, 326, 300, 178, 131, 119, 117, 105, 91, 85, 69, 67.

Reference Example 11

(5EZ,13E)-(9α,11α)-6,9-methano-11-(tetrahydropyran-2-yloxy)-15-oxo-15-cyclopentyl-16,17,18,19,20-pentanor-prosta-5,13-dienoic acid methyl ester

Proceeding as described in Example 1, from 90 mg of the 15-hydroxyprosta-5,13-dienoic acid methyl ester compound (prepared in Reference Example 10), there were obtained 79 mg of the title compound as crude product having the following physical characteristic:

TLC (ethyl acetate:cyclohexane=1:2): Rf=0.49.

Reference Example 12

(5EZ,13E)-(9α,11α)-6,9-methano-11-hydroxy-15-oxo-15-cyclopentyl-16,17,18,19,20-pentanorprosta-5,13-dienoic acid methyl ester

A mixture of 79 mg of the 11-(tetrahydropyran-2-yloxy)prosta-5,13-dienoic acid methyl ester compound (prepared in Reference Example 11), 2 ml of a 65% (v/v) aqueous solution of acetic acid and 0.3 ml of tetrahydrofuran was stirred for 1.5 hours at 50°C and thereto was added ice-water and the mixture was extracted with ethyl acetate. The extract was washed with a saturated aqueous solution of sodium bicarbonate and a saturated aqueous solution of sodium chloride, successively, dried over anhydrous magnesium sulphate and concentrated under reduced pressure. The residue was purified by column chromatography on silica gel (eluent; a mixture of methylene chloride and diethyl ether) to give 57 mg of the title compound having the following physical characteristics:

TLC (ethyl acetate:cyclohexane=1:2, twice developed): Rf=0.30 and 0.34;

NMR: δ=6.72 (1H, dd), 6.10 (1H, d), 5.5-4.92 (1H, m), 3.6 (3H, s);

IR: ν=3450, 2960, 2875, 1740, 1690, 1660, 1620, 1450, 1435 $cm^{-1}$;

Mass: m/e=360($M^+$), 342, 316, 311, 273, 245, 179, 162, 149, 147, 131, 129, 119, 107, 105, 97, 91, 79, 69.

Reference Example 13

(5E,13E)-(9α,11α)-6,9-methano-11-hydroxy-15-oxo-15-cyclopentyl-16,17,18,19,20-pentanorprosta-5,13-dienoic-acid methyl ester and the corresponding (5Z)-isomer

2.3 g of the (5EZ)-mixture (prepared in Reference Example 12) were purified by chromatography on a Lobar® column. (Art 10401: "Lobar" is a registered Trade Mark of Merck & Co., Inc.) [eluent; a mixture of cyclohexane and ethyl acetate (4:1 → 3:1)] to give 1.51 g of the title compound [(5E)-isomer], 310 mg of the corresponding (5Z)-isomer and 307 mg of the mixture thereof, having the following physical characteristics:

(a) the title compound [(5E)-isomer]:

TLC (ethyl acetate:cyclohexane=1:2, twice developed): Rf=0.30;

NMR: δ=6.72 (1H, dd), 6.10 (1H, d), 5.5-4.92 (1H, m), 3.6 (3H, s);

IR: ν=3450, 2960, 2875, 1740, 1690, 1660, 1620, 1450, 1435 $cm^{-1}$;

Mass: m/e=360($M^+$), 342, 316, 311, 273, 245, 179, 162,

149, 147, 131, 129, 119, 107, 105, 97, 91, 79, 69.

(b) the corresponding (5Z)-isomer:

TLC (ethyl acetate:cyclohexane=1:2, twice developed); Rf=0.34.

Reference Example 14

(5E,13E)-(9α,11α,15α)-6,9-methano-11,15-dihydroxy-15-cyclopentyl-16,17,18,19,20-pentanorprosta-5,13-dienoic acid methyl ester and the corresponding 15β-hydroxy isomer

Under an atmosphere of argon, to a solution of 17.2 g of 2,6-di-tert-butyl-p-cresol in 90 ml of toluene were added dropwise 23.6 ml of a 1.76 M solution of diisobutylaluminium hydride in toluene under cooling with ice and the mixture was stirred for 30 minutes at room temperature and then cooled to -78°C. To the solution thus obtained was added dropwise slowly a solution of 1.51 g of the 15-oxo compound [prepared in Reference Example 13(a)] in 10 ml of toluene at -78°C. After completion of adding dropwise, a cooling bath was removed and the temperature of the reaction mixture was raised to -20°C over a period of ca. 30 minutes with stirring the mixture and further to -13°C over a period of ca. 40 minutes. The reaction mixture was quenched

by adding 16 ml of water and stirred for 30 minutes at room temperature. The precipitate was filtered off and the filtrate was concentrated under reduced pressure. The residue was purified by column chromatography on silica gel (eluent; a mixture of methylene chloride and ethyl acetate, and ethyl acetate, successively) to give 1.14 g of the title compound (15α-hydroxy compound) and 203 mg of the corresponding 15β-hydroxy isomer, both being as white crystal, having the following physical characteristics:

(a) the title compound (15α-hydroxy isomer):

Melting point: 77-79°C (recrystallized from a mixture of ethyl acetate and n-hexane);

TLC (ethyl acetate:cyclohexane=2:1): Rf=0.26;

NMR: δ=5.87-4.83 (3H, m), 4.27-3.4 (2H, m), 3.6 (3H, s);

IR (KBr tablet method): ν=3380, 2950, 2875, 1740, 1450, 1430, 1315, 1250, 1195, 1170, 1130, 1085, 1020, 970 $cm^{-1}$;

Mass: m/e=344, 326, 313, 300, 275, 232, 179.

(b) the corresponding 15β-hydroxy isomer:

TLC (ethyl acetate:cyclohexane=2:1): Rf=0.38.

Reference Example 15

(5E,13E)-(9α,11α,15α)-6,9-methano-11,15-dihydroxy-15-cyclopentyl-16,17,18,19,20-pentanorprosta-5,13-dienoic acid [i.e. (5E)-15-cyclopentyl-16,17,18,19,20-pentanor-

6,9-methano-$PGI_2$]

A mixture of 1.08 g of the methyl ester compound [prepared in Reference Example 14(a)], 15 ml of a 5% aqueous solution of potassium hydroxide and 15 ml of ethanol was stirred for 20 minutes at 45°C and was then adjusted to pH 3 to 4 with 1N hydrochloric acid under cooling with ice. The reaction mixture was diluted with ethyl acetate, washed with water and a saturated aqueous solution of sodium chloride, successively, dried over anhydrous magnesium sulphate and concentrated under reduced pressure to give 1.01 g of the title compound as crude crystal. The crude crystal thus obtained was recrystallized from a mixture of ethyl acetate and cyclohexane (1:2) to give 955 mg of the title compound as white powder having the following physical characteristics:

Melting point: 102-103°C;

TLC [ethyl acetate:cyclohexane=3:1 (containing 2% acetic acid)]: Rf=0.23;

NMR: δ=6.55-4.95 (6H, m), 4.2-3.35 (2H, m);

IR (KBr tablet method): ν=3350, 2950, 2875, 2650, 1710, 1450, 1430, 1410, 1375, 1250, 1220, 1130, 1080, 1020, 970 $cm^{-1}$;

Mass: m/e=330, 312, 286, 261, 243, 217, 165, 91, 69, 41.

Example 4

(E)-3αß-acetoxy-6-syn-(3α-hydroxy-3-cyclopentylprop-1-enyl)-7-anti-(tetrahydropyran-2-yloxy)-cis-bicyclo[3.3.0]-octane and the corresponding 3ß-hydroxy isomer

Under an atmosphere of argon, to a suspension of 1.125 g of lithium aluminium hydride in 55 ml of tetrahydrofuran was added dropwise a solution of 1.48 ml of ethanol diluted in 15 ml of tetrahydrofuran over a period of 5 minutes at 10°C, and further thereto was added dropwise a solution of 7.264 g of (S)-2,2'-dihydroxy-1,1'-binaphthyl in 27 ml of tetrahydrofuran over a period of 10 minutes at 10°C and the mixture was stirred for 20 minutes at room temperature to give a suspension of lithium (S)-1,1'-binaphthyl-2,2'-dioxy-ethoxyaluminium hydride in tetrahydrofuran (it is a reagent for selective reduction). After cooling the reagent thus obtained to -78°C, thereto was added dropwise a solution of 1.65 g of the 6-(3-oxo-3-cyclopentylprop-1-enyl)-bicyclo[3.3.0]octane compound (prepared in Example 2) in 15 ml of tetrahydrofuran over a period of 10 minutes and the mixture was stirred for one hour at the same temperature. Methanol was added dropwise to the reaction mixture to decompose an excess amount of the reagent and then the temperature of the mixture was raised to 0°C. The reaction mixture was adjusted to pH

7 to 8 with 1N hydrochloric acid and filtered. The filtrate was washed with a saturated aqueous solution of sodium chloride, dried over anhydrous magnesium sulphate and concentrated under reduced pressure. The residue was purified by column chromatography on silica gel (eluent; a mixture of methylene chloride and ethyl acetate, and a mixture of ethyl acetate and cyclohexane, successively) to give 1.53 g of the title compound (3α-hydroxy isomer) and 38 mg of the corresponding 3β-hydroxy isomer having the following physical characteristics:

(a) the title compound (3α-hydroxy isomer):

TLC (ethyl acetate:cyclohexane=1:2): Rf=0.20;

NMR: δ=5.58 (2H, m), 5.12 (1H, m), 4.65 (1H, m), 3.94-3.65 (3H, m), 3.46 (1H, m), 2.03 (3H, s);

IR: ν=3460, 1735, 1440, 1375, 1245, 1120, 1075, 1020, 975 $cm^{-1}$;

Mass: m/e=290, 272, 248, 230, 221, 212, 196, 186, 161, 149, 136, 85, 69, 67.

(b) the corresponding 3β-hydroxy isomer:

TLC (ethyl acetate:cyclohexane=1:2): Rf=0.22;

Reference Example 16

(E)-3aβ-acetoxy-6-syn-[3α-(tetrahydropyran-2-yloxy)-3-cyclopentylprop-1-enyl]-7-anti-(tetrahydropyran-2-yloxy)-cis-bicyclo[3.3.0]octane

Proceeding as described in Reference Example 1, from 1.57 g of the 6-(3α-hydroxy-3-cyclopentylprop-1-enyl)-bicyclo[3.3.0]octane compound [prepared in Example 4(a)], there were obtained 2.0 g of the title compound as crude product having the following physical characteristic:

TLC (ethyl acetate:cyclohexane=1:2): Rf=0.48.

Reference Example 17

(E)-6-syn-[3α-(tetrahydropyran-2-yloxy)-3-cyclopentyl-prop-1-enyl]-7-anti-(tetrahydropyran-2-yloxy)-cis-bicyclo[3.3.0]octan-3αβ-ol

Proceeding as described in Reference Example 7, from 2.0 g of the 3-acetoxy-bicyclo[3.3.0]octane compound (prepared in Reference Example 16), there were obtained 1.8 g of the title compound as crude product having the following physical characteristic:

TLC (ethyl acetate:cyclohexane=1:2): Rf=0.19 and 0.23.

Reference Example 18

(E)-6-syn-[3α-(tetrahydropyran-2-yloxy)-3-cyclopentyl-prop-1-enyl]-7-anti-(tetrahydropyran-2-yloxy)-cis-bicyclo[3.3.0]octan-3-one

Proceeding as described in Example 1, from 1.8 g of the bicyclo[3.3.0]octan-3-ol compound (prepared in Reference Example 17), there were obtained 1.5 g of the title compound having the following physical characteristics:

TLC (ethyl acetate:cyclohexane=1:2): Rf=0.34;

NMR: δ=5.7-5.2 (2H, m), 4.65 (2H, m), 4.2-3.1 (6H, m);

IR: ν=1740, 980 $cm^{-1}$;

Mass: m/e=363, 331, 330, 279, 246, 228, 202, 177, 165, 152, 101, 94, 85, 67. 57.

Reference Example 19

(5EZ,13E)-(9α,11α,15α)-6,9-methano-11,15-bis(tetrahydropyran-2-yloxy)-15-cyclopentyl-16,17,18,19,20-pentanorprosta-5,13-dienoic acid

Proceeding as described in Reference Example 9, from 1.47 g of the bicyclo[3.3.0]octan-3-one compound (prepared in Reference Example 18), there were obtained 1.554 g of the title compound (777 mg of the almost pure 5E-isomer, 525 mg rich in 5E-isomer and 252 mg rich in 5Z-isomer) having the following physical characteristics:

TLC (ethyl acetate:cyclohexane=1:2): Rf=0.24 and 0.30;

NMR: δ=5.70-5.13 (3H, m), 4.65 (2H, m), 3.96-3.33 (6H, m);

IR: ν=1730, 1710, 1445, 1335, 1200, 1120, 1075, 1015, 975, 910, 865, 810, 755 $cm^{-1}$;

Mass: m/e=414, 396, 370, 363, 330, 312, 286, 279, 261, 218, 164, 149, 105, 91, 85, 67, 57, 55.

Reference Example 20

(5EZ,13E)-(9α,11α,15α)-6,9-methano-11,15-dihydroxy-15-cyclopentyl-16,17,18,19,20-pentanorprosta-5,13-dienoic acid

A mixture of 525 mg of the 11,15-bis(tetrahydropyran-2-yloxy)prosta-5,13-dienoic acid compound rich in 5E-isomer (prepared in Reference Example 19), 5 ml of 1N hydrochloric acid and 10 ml of tetrahydrofuran was stirred for 7 hours at room temperature, and further for 16 hours at 0°C. The reaction mixture was diluted with ethyl acetate and washed with water and a saturated aqueous solution of sodium chloride, successively, dried over anhydrous magnesium sulphate and concentrated under reduced pressure. The residue was purified by column chromatography on silica gel (eluent; a mixture of ethyl acetate and cyclohexane) to give 244 mg of the title compound as white crystal having the following physical characteristics:

TLC [ethyl acetate:cyclohexane=3:1 (containing 2% acetic acid), twice developed]: Rf=0.44 and 0.49;

NMR: δ=6.55-4.95 (6H, m), 4.2-3.35 (2H, m);

IR (KBr tablet method): ν=3350, 2950, 2875, 2650, 1710, 1450, 1430, 1410, 1375, 1250, 1220, 1130, 1080, 1020, 970 $cm^{-1}$;

Mass: m/e=330, 312, 286, 261, 243, 217, 165, 91, 69, 41.

Reference Example 21

(5E,13E)-(9α,11α,15α)-6,9-methano-11,15-dihydroxy-15-cyclopentyl-16,17,18,19,20-pentanorprosta-5,13-dienoic acid [i.e. (5E)-15-cyclopentyl-16,17,18,19,20-pentanor-6,9-methano-$PGI_2$]

To a solution of 244 mg of the (5EZ)-mixture (prepared in Reference Example 20) in 7.5 ml of acetone was added 0.14 ml of dicylohexylamine and after adequate dissolution, the solution was allowed to stand for 18 hours at room temperature, and the precipitated crystals were filtered and washed with acetone. The crystals obtained were dissolved in a 0.5N aqueous solution of potassium bisulphate and extracted with ethyl acetate. The extract was washed with water and a saturated aqueous solution of sodium chloride, successively and concentrated under reduced pressure to give 145 mg of the white crystals. The crystals thus obtained were recrystallized from a mixture of ethyl acetate and

cyclohexane (1:2) to give 114 mg of the title compound as white crystal having the same physical characteristics (provided that the Rf value of TLC shows 0.44) as those of the product prepared in Reference Example 20.

Reference Example 22

(5E,13E)-(9α,11α,15α)-6,9-methano-11,15-dihydroxy-15-cyclopentyl-16,17,18,19,20-pentanorprosta-5,13-dienoic acid [i.e. (5E)-15-cyclopentyl-16,17,18,19,20-pentanor-6,9-methano-$PGI_2$]

Proceeding as described in Reference Example 20, from 757 mg of the 11,15-bis(tetrahydropyran-2-yloxy)prosta-5,13-dienoic acid compound [almost pure (5E)-isomer, prepared in Reference Example 19], there were obtained 363 mg of almost pure title compound as white crystals. The crystals thus obtained were recrystallized from a mixture of ethyl acetate and cyclohexane (1:2) to give 285 mg of the title compound as white crystal having the same physical characteristics as those of the product prepared in Reference Example 21.

Reference Example 23

(5E,13E)-(9α,11α,15α)-6,9-methano-11,15-bis(tetrahydropyran-2-yloxy)-15-cyclopentyl-16,17,18,19,20-pentanorprosta-5,13-dienoic acid methyl ester

A mixture of 1.78 g of the almost pure 5E-isomer prepared as described in Reference Example 19 [(5E,13E)-(9α,11α,15α)-6,9-methano-11,15-bis(tetrahydropyran-2-yloxy)-15-cyclo-pentyl-16,17,18,19,20-pentanorprosta-5,13-dienoic acid], 0.95 g of potassium carbonate, 1.07 ml of methyl iodide and 25 ml of acetone was refluxed gently for 1.5 hours. The reaction mixture was cooled and then concentrated under reduced pressure to one-third the original volume of acetone. The residual solution thus obtained was diluted with ethyl acetate and washed with water, a saturated aqueous solution of ammonium chloride and a saturated aqueous solution sodium chloride, successively, dried over anhydrous magnesium sulphate and concentrated under reduced pressure to give 1.75 g of the title compound as crude product having the following physical characteristics:

TLC (ethyl acetate:cyclohexane=1:2): Rf=0.52;

NMR: δ=5.65-5.10 (3H, m), 4.70 (2H, m), 4.00-3.30 (9H, m);

IR: ν=1735, 1435, 1350, 1250, 1200, 1130, 1080, 1020, 980 $cm^{-1}$;

Mass: m/e=428, 384, 344, 326, 300.

Reference Example 24

(5E,13E)-(9α,11α,15α)-6,9-methano-11,15-dihydroxy-15-cyclopentyl-16,17,18,19,20-pentanorprosta-5,13-dienoic acid methyl ester

A mixture of 1.75 g of the 11,15-bis(tetrahydropyran-2-yloxy)prosta-5,13-dienoic acid methyl ester compound (prepared in Reference Example 23), ca. 12 mg of p-toluenesulphonic acid and 22 ml of methanol was stirred for 4 hours at room temperature. After addition of one ml of triethylamine, the reaction mixture was concentrated under reduced pressure. The residue was purified by column chromatography on silica gel (eluent; a mixture of ethyl acetate and cyclohexane, and ethyl acetate, successively) to give 1.0 g of the title compound as white solid having the same physical characteristics as those of the product prepared in Reference Example 14(a).

Reference Example 25

(5E,13E)-(9α,11α,15α)-6,9-methano-11,15-dihydroxy-15-cyclopentyl-16,17,18,19,20-pentanorprosta-5,13-dienoic acid [i.e. (5E)-15-cyclopentyl-16,17,18,19,20-pentanor-

6,9-methano-$PGI_2$]

Proceeding as described in Reference Example 15, from 967 mg of the methyl ester compound (prepared in Reference Example 24), there were obtained 822 mg of the title compound having the same physical characteristics as those of the product prepared in Reference Example 15.

CLAIMS FOR CONTRACTING STATES: BE, CH DE, FR, GB, IT, LI, LU, NL, SE.

1. A compound of the general formula:

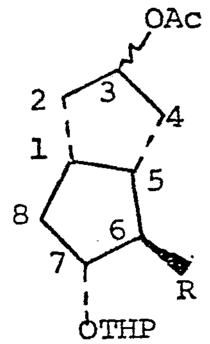

VII

wherein Ac represents an acetyl group, THP represents a tetrahydropyran-2-yl group and R represents a formyl group or a group of the general formula:

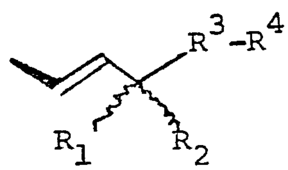

VIII

(in which one of $R^1$ and $R^2$ represents a hydroxy group and the other represents a hydrogen atom, or $R^1$ and $R^2$ together represent an oxo group, $R^3$ represents a single bond or a straight- or branched-chain alkylene group of 1 to 5 carbon atoms and $R^4$ represents a straight- or branched-chain alkyl group of 1 to 8 carbon atoms, a straight- or branched-chain alkenyl or alkynyl group of 2 to 8 carbon atoms, a cycloalkyl group of 4 to 7 carbon atoms either unsubstituted or substituted by at least one straight- or branched-chain alkyl group of 1 to 8 carbon atoms or represents a phenyl

group or phenoxy group either unsubstituted or substituted by at least one halogen atom, trifluoromethyl group or straight- or branched-chain alkyl group of 1 to 4 carbon atoms, and the double bond in general formula VIII is E; with the proviso that when $R^3$ represents a single bond, $R^4$ does not represent a substituted or unsubstituted phenoxy group).

2. A compound according to claim 1 wherein $R^3$ represents a single bond or a methylene or ethylene group.

3. A compound according to claim 2 wherein $R^4$ represents a cycloalkyl group of 4 to 7 carbon atoms either unsubstituted or substituted by at least one straight- or branched-chain alkyl group of 1 to 8 carbon atoms.

4. A compound according to claim 1 wherein the grouping $-R^3-R^4$ represents a cyclopentyl group.

5. A compound according to any one of the preceding claims wherein, in general formula VIII depicted in claim 1, one of $R^1$ and $R^2$ represents a hydroxy group in α-configuration and the other represents a hydrogen atom.

6. A compound according to claim 1. which is (E)-3αß-acetoxy-6-syn-(3-oxo-3-cyclopentylprop-1-enyl)-7-anti-(tetrahydropyran-2-yloxy)cis-bicyclo[3.3.0]-octane.

7. A compound according to claim 1 which is (E)-3αß-acetoxy-6-syn-(3-hydroxy-3-cyclopentylprop-1-enyl)-7-anti-(tetrahydropyran-2-yloxy)cis-bicyclo[3.3.0]-octane.

8. A process for the preparation of a compound according to claim 1, which comprises:

(a) the oxidation of a compound of the general formula:

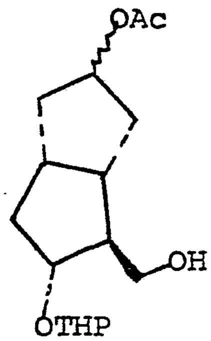

XVI

(wherein Ac and THP are as defined in claim 1) to obtain a compound of the general formula:

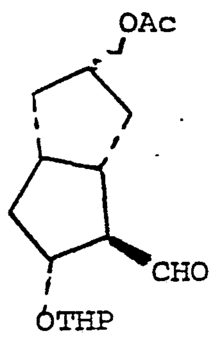

VIIC

(wherein Ac and THP are as defined in claim 1), followed, if desired, by

(b) the reaction of the compound of general formula VIIC, wherein THP and Ac are as defined in claim 1, with a sodium derivative of a dialkyl phosphonate of the general formula:

$$(R^5O)_2\underset{\underset{O}{\|}}{P}\,CH_2\,\underset{\underset{O}{\|}}{C}-R^3-R^4$$

(wherein $R^5$ represents an alkyl group of 1 to 4 carbon atoms and the other symbols are as defined in claim 1) or with a phosphorane compound of the general formula:

$$(R^6)_3\ P = CH\underset{\underset{O}{\|}}{C}-R^3-R^4$$

(wherein $R^6$ represents a phenyl group unsubstituted or substituted by at least one alkyl group of 1 to 4 carbon atoms or represents an alkyl group of 1 to 6 carbon atoms or represents a cyclohexyl group, and the other symbols are as defined in claim 1) to obtain a compound of the general formula:

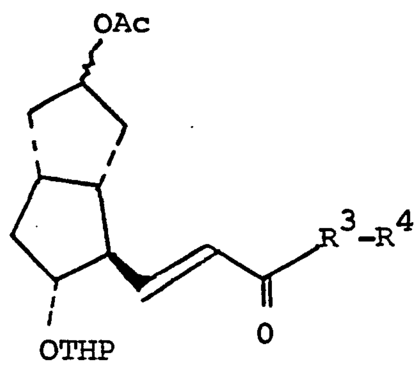

VIIB

(wherein the various symbols are as defined in claim 1), followed, if desired, by

(c) the reduction of a compound of the general formula VIIB depicted in claim 9, (wherein the various symbols are as defined in claim 1) to obtain a compound of the general formula:

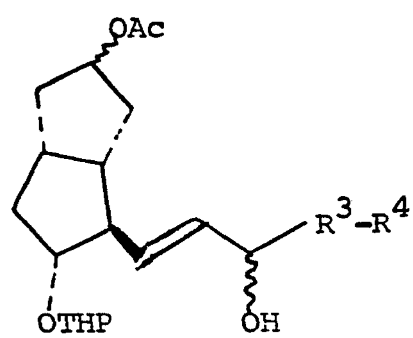

VIIA

wherein the various symbols are as defined in claim 1.

CLAIMS FOR CONTRACTING STATE : AT

1. A process for the preparation of a compound of the general formula:

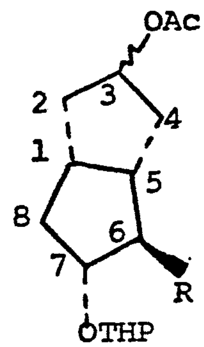

VII

wherein Ac represents an acetyl group, THP represents a tetrahydropyran-2-yl group and R represents a formyl group or a group of the general formula:

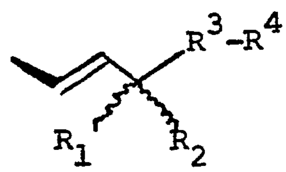

VIII

(in which one of $R^1$ and $R^2$ represents a hydroxy group and the other represents a hydrogen atom, or $R^1$ and $R^2$ together represent an oxo group, $R^3$ represents a single bond or a straight- or branched-chain alkylene group of 1 to 5 carbon atoms and $R^4$ represents a straight- or branched-chain alkyl group of 1 to 8 carbon atoms, a straight- or branched-chain alkenyl or alkynyl group of 2 to 8 carbon atoms, a cycloalkyl group of 4 to 7 carbon atoms either unsubstituted or substitute by at least one straight- or branched-chain alkyl group

of 1 to 8 carbon atoms or represents a phenyl group or phenoxy group either unsubstituted or substituted by at least one halogen atom, trifluoromethyl group or straight- or branched-chain alkyl group of 1 to 4 carbon atoms, and the double bond in general formula VIII is E; with the proviso that when $R^3$ represents a single bond, $R^4$ does not represent a subsituted or unsubstituted phenoxy group), which process comprises:

(a) the oxidation of a compound of the general formula:

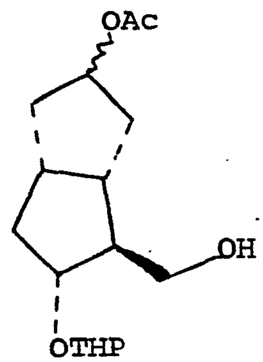

XVI

(wherein Ac and THP are as hereinbefore defined) to obtain a compound of the general formula:

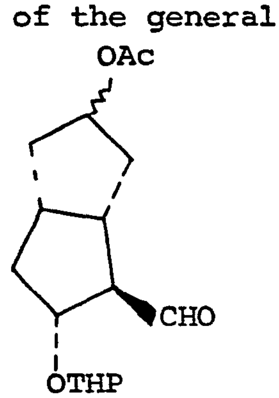

VIIC

(wherein Ac and THP are as hereinbefore defined) followed, if desired, by

(b) the reaction of a compound of general formula VIIC wherein THP and Ac are as hereinbefore defined with a sodium

derivative of a dialkyl phosphonate of the general formula:

$$(R^5O)_2\underset{\underset{O}{\|}}{P}\,CH_2\,\underset{\underset{O}{\|}}{C}-R^3-R^4$$

(wherein $R^5$ represents an alkyl group of 1 to 4 carbon atoms and the other symbols are as hereinbefore defined) or with a phosphorane compound of the general formula:

$$(R^6)_3\ P{=}CH\underset{\underset{O}{\|}}{C}-R^3-R^4$$

(wherein $R^6$ represents a phenyl group unsubstituted or substituted by at least one alkyl group of 1 to 4 carbon atoms or represents an alkyl group of 1 to 6 carbon atoms or represents a cyclohexyl group, and the other symbols are as hereinbefore defined) to obtain a compound of the general formula:

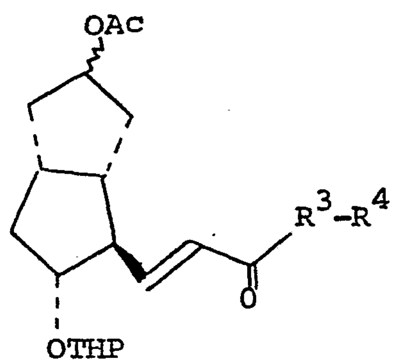

VIIB

(wherein the various symbols are as hereinbefore defined) followed, if desired, by

(c) the reduction of a compound of the general formula VIIB (wherein the various symbols are as hereinbefore defined) to obtain a compound of the general formula:

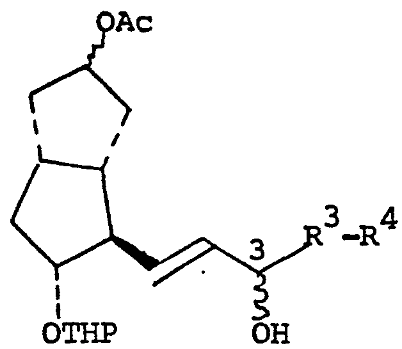

VIIA

wherein the various symbols are as hereinbefore defined.

2. A process according to claim 1 wherein $R^3$ represents a single bond or a methylene or ethylene group.

3. A process according to claim 2 wherein $R^4$ represents a cycloalkyl group of 4 to 7 carbon atoms either unsubstituted or substituted by at least one straight- or branched-chain alkyl group of 1 to 8 carbon atoms.

4. A process according to claim 1 wherein the grouping $-R^3-R^4$ represents a cyclopentyl group.

5. A process according to claim 1 wherein, in general formula VIIA, the hydroxy group attached to the 3-position is in α-configuration.

European Patent Office

**0105651**

# EUROPEAN SEARCH REPORT

Application number

EP 83 30 5422

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| D,X | GB-A-2 014 143 (UPJOHN)<br>* Whole document and particularly page 70, example 44 *<br>----- | 1-8 | C 07 D 309/12 //<br>C 07 C 177/00 |
| | | | TECHNICAL FIELDS SEARCHED (Int. Cl. 3) |
| | | | C 07 D 309/00 |

The present search report has been drawn up for all claims

| Place of search | Date of completion of the search | Examiner |
|---|---|---|
| THE HAGUE | 08-12-1983 | ALLARD M.S. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons

& : member of the same patent family, corresponding document

EPO Form 1503. 03.82